# EUROPEAN PATENT SPECIFICATION

(11) **EP 3 354 746 B1**
(45) Date of publication and mention of the grant of the patent: **29.05.2019**
(21) Application number: 17153689.9
(22) Date of filing: 30.01.2017
(51) Int. Cl.: C12Q 1/6869, C12Q 1/6809

(54) **NOVEL SPIKE-IN OLIGONUCLEOTIDES FOR NORMALIZATION OF SEQUENCE DATA**
NEUARTIGE SPIKE-IN-OLIGONUKLEOTIDE ZUR NORMALISIERUNG VON SEQUENZDATEN
NOUVEAUX OLIGONUCLÉOTIDES INSÉRÉS POUR NORMALISER LES DONNÉES DE SÉQUENCE

(43) Date of publication of application: 01.08.2018
(73) Proprietor: Gregor Mendel Institute of Molecular Plant Biology GmbH, 1030 Vienna (AT)
(72) Inventor: NODINE, Michael Douglas, 1040 Vienna (AT)
(74) Representative: Loidl, Manuela Bettina

(56) References cited:
- WO-A1-2014/082032
- WO-A1-2015/118513
- WO-A1-2016/001736
- WO-A1-2016/007951
- MAURO D. LOCATI ET AL: "Improving small RNA-seq by using a synthetic spike-in set for size-range quality control together with a set for data normalization", NUCLEIC ACIDS RESEARCH, vol. 43, no. 14, 18 August 2015 (2015-08-18), pages e89-e89, XP055386708, ISSN: 0305-1048, DOI: 10.1093/nar/gkv303
- N. FAHLGREN ET AL: "Computational and analytical framework for small RNA profiling by high-throughput sequencing", RNA, vol. 15, no. 5, 24 March 2009 (2009-03-24) , pages 992-1002, XP055386707, US ISSN: 1355-8382, DOI: 10.1261/rna.1473809
- MICHAEL A QUAIL ET AL: "SASI-Seq: sample assurance Spike-Ins, and highly differentiating 384 barcoding for Illumina sequencing", BMC GENOMICS, BIOMED CENTRAL LTD, LONDON, UK, vol. 15, no. 1, 7 February 2014 (2014-02-07), page 110, XP021182710, ISSN: 1471-2164, DOI: 10.1186/1471-2164-15-110
- L. JIANG ET AL: "Synthetic spike-in standards for RNA-seq experiments", GENOME RESEARCH, vol. 21, no. 9, 4 August 2011 (2011-08-04) , pages 1543-1551, XP055152443, ISSN: 1088-9051, DOI: 10.1101/gr.121095.111
- DOMINIK BUSCHMANN ET AL: "Toward reliable biomarker signatures in the age of liquid biopsies - how to standardize the small RNA-Seq workflow", NUCLEIC ACIDS RESEARCH, vol. 44, no. 13, 17 June 2016 (2016-06-17) , pages 5995-6018, XP055386895, ISSN: 0305-1048, DOI: 10.1093/nar/gkw545

## Description

### Field of the invention

The invention relates to novel spike-in oligonucleotides for use in quantitative normalization of nucleotide sequence data. The invention provides sets comprising at least two subsets, each subset comprising a plurality of single stranded nucleic acid molecules, wherein each single stranded nucleic acid molecule comprises a 5'phosphate, a sequence of at least 3 randomized nucleotides, a core sequence of at least 8 nucleotides not complementary to a target sequence, a sequence of at least 3 randomized nucleotides, and a 3'modification. The nucleic acid molecules of the subsets differ in at least one nucleotide of the core nucleotide sequence. The invention also relates to the generation of a library containing the sets and to reference values in nucleotide sequencing and a method for determining the amount of target sequences in a sample.

### Background of the invention

Since first introduced to the market in 2005, next-generation sequencing (NGS), massively parallel or deep sequencing technologies, have had a tremendous impact on genomic research. The next-generation technologies have been used for standard sequencing applications, such as genome sequencing and resequencing, and for novel applications previously unexplored by Sanger sequencing. All NGS platforms perform sequencing of millions of small fragments of DNA in parallel and thus offer dramatic increases in cost-effective sequence throughput, albeit at the expense of read lengths.

Small RNA NGS library construction takes smallRNA as input and results in a library of corresponding cDNAs. This results in short sequences of nucleotides, i.e. reads. In order to deduce which RNA molecules are present in the original sample, the reads are mapped or aligned onto a reference genome or transcriptome, or are *de novo* assembled based on sequence overlaps. Bioinformatics analyses are used to piece together these fragments by mapping the individual reads to the reference genome.

Data from high-throughput small RNA sequencing (sRNA-Seq) experiments are typically normalized and reported in relative terms such as reads per million genome-matching reads (RPMs)¹. Relative normalization works well if it is assumed that the sRNA sub-populations have equal proportions across the different tissue types being profiled. However, this assumption is often invalid because sRNA populations are frequently dynamic across different tissue types and in various mutant backgrounds²⁻⁷. For example, based on identical starting material in miRNA quantification, the analyses demonstrated that alternative methods in cDNA construction resulted in entirely different miRNA expression level profiles³³. Enzymes involved in RNA end-modification are obvious candidates for causing bias in relative expression levels, however steps of reverse transcription and PCR amplification are also likely to display template preferences thereby favoring the amplification of some RNAs over others. Therefore, the standard practice of comparing relatively normalized sRNA-Seq values can produce misleading results. In contrast, absolute normalization of sRNA-Seq data should enable accurate comparisons of small RNA levels in different cell types, mutant tissues or disease states on a genome-wide scale. However, previous attempts to use exogenous sRNA oligonucleotides for absolute normalization of sRNA-Seq data had variable success⁸⁻¹⁰ and thus have not been widely used. Locati et al. used two separate sets of synthetic RNA spike-ins, consisting of 11 and 19 oligoribonucleotides respectively, for monitoring size-selection and for performing data normalization in sRNA-seq (Locati et al. Nucleic Acids Res. 2015 Aug 18;43(14)). The varying sequencing efficiencies of specific sRNA spike-ins are most likely due to their inherent properties, such as variable secondary structures and RNA-adapter cofolding that influence their ligation efficiency during sRNA-Seq library construction¹¹⁻¹³. For example, previously an additional correction factor was required to scale individual sRNA spike-in amounts to account for the non-linear relationships between the molar amount of exogenous sRNA spike-ins added to a sample and the corresponding number of reads sequenced¹⁰.

Thus although NGS is a powerful tool for novel gene discovery and fine-tuned transcriptional profiling, there are issues which are still unsolved using the currently available techniques:
- Ligation of adaptors to small RNAs is biased due to secondary structure and potentially nucleotide biases; therefore, certain small RNA sequences may be over- or under-represented in final datasets. One solution has been to use adaptors, which are ligated to small RNAs to generate "libraries" of millions of cloned small RNAs, with randomized terminal positions. Although these help reduce bias, it is unknown how much biases still exists which would be important for any small RNA-Seq experiment.
- RNA-Seq only reports relative numbers of small RNA levels (e.g. reads per million genome-matching reads) rather than absolute numbers (e.g. numbers of molecules); therefore, it is impossible to compare small RNA-Seq data from various tissues with dynamic small RNA populations (which is often the case), as well as between mutants that alter these populations (e.g. known and unknown mutants that perturb different small RNA biogenesis factors).

Normalization of high-throughput RNA and DNA sequencing data is required to compare RNA or DNA, specifically sRNA levels across different samples. Commonly used relative normalization approaches can cause erroneous conclusions due to fluctuating RNA populations, e.g. small RNA populations between tissues. Thus there is a need for methods and products that allow a more accurate and reproducible assessment, specifically for absolute normalization of sequence data.

### Summary of the invention

The problems are solved by the subject of the present invention.

Provided herein is a set comprising at least two subsets of single stranded nucleic acid molecules, each nucleic acid molecule comprising from the 5'to 3'direction:
a) a 5'phosphate, optionally a monophosphate, diphosphate or triphosphate,
b) a sequence of at least 3 randomized nucleotides,
c) a core sequence of at least 8 nucleotides not complementary to a target sequence,
d) a sequence of at least 3 randomized nucleotides, and
e) a 3'modification, optionally 2'-O-methylation or hydroxylation,
wherein each subset comprises a plurality of single stranded nucleic acid molecules which have an identical core nucleotide sequence and different randomized nucleotides, and wherein the nucleic acid molecules of each subset differ in at least one nucleotide of the core nucleotide sequence.

According to an embodiment there is provided a set comprising at least two subsets of single stranded nucleic acid molecules, each nucleic acid molecule comprising from the 5' to 3' direction:
a) a 5'phosphate, optionally a monophosphate, diphosphate or triphosphate,
b) a sequence of at least 3 randomized nucleotides,
c) a core sequence of at least 8 nucleotides which sequence contains two or more mismatches compared to a target sequence,
d) a sequence of at least 3 randomized nucleotides, and
e) a 3'modification, optionally 2'-O-methylation or hydroxylation,
wherein each subset comprises a plurality of nucleic acid molecules having an identical core nucleotide sequence and different randomized nucleotides, and wherein the nucleic acid molecules of each subset differ in at least one nucleotide of the core nucleotide sequence.

The present invention specifically provides a set of sRNA spike-in oligonucleotides (sRNA spike-ins) that enable absolute quantitative normalization of sRNA-Seq data across independent experiments, as well as the genome-wide estimation of sRNA: mRNA stoichiometries when used together with mRNA spike-in oligonucleotides.

Absolute quantification of the number of small RNA molecules in the sample not only enables comparison between small RNA-Seq datasets, but also between small RNA-Seq and mRNA-Seq datasets that also use exogenous spike-ins such as the ERCC spike-in mixes from Life Tech.

The small RNA spike-in sets of the invention not only serve as useful internal controls for sRNA-Seq experiments requiring only 1-2% of the alignable reads for subsequent analyses, but can also be used to normalize sRNA-Seq data from different sources, e.g. different treatments, tissue types or research groups. Moreover, they enable the absolute quantification of sRNA molecules, which can be essential for accurate comparisons, as well as genome-wide estimations of precursor:sRNA and sRNA:target stoichiometries, which are important for understanding these relationships on a molecular scale. The inventive sRNA spike-in set could also be used to assess and improve upon cloning biases that exist in various sRNA-Seq library generation protocols.

Said sRNA spike-ins can also be used in combination with commercially available mRNA spike-in oligonucleotide mixes to compare values generated by sRNA-Seq and mRNA-Seq.

According to a specific embodiment of the invention, there is provided a set comprising at least two subsets of single stranded nucleic acid molecules, each nucleic acid molecule from 5' to 3' consisting of:
a) a 5'phosphate, optionally a monophosphate, diphosphate or triphosphate,
b) a sequence of at least 3 randomized nucleotides,
c) a core sequence of at least 8 nucleotides containing two or more mismatches compared to a target sequence,
d) a sequence of at least 3 randomized nucleotides, and
e) a 3'modification, optionally 2'-O-methylation or hydroxylation,
wherein each subset comprises a plurality of nucleic acid molecules having an identical core nucleotide sequence and different randomized nucleotide sequences, and wherein the nucleic acid molecules of each subset differ in at least one nucleotide of the core nucleotide sequence.

In one embodiment, the core sequence is not complementary to the target sequence.

According to an embodiment of the invention, the randomized nucleotides are any one of A, C, G, U. or A, C, G, T.

According to a specific embodiment of the invention, the plurality of nucleic acid molecules comprises randomized nucleotide sequences containing all four nucleotide combinations of A, C, G, U or A, C, G, T.

According to an embodiment of the invention, the nucleic acid molecule is an RNA molecule, specifically mimicking a small RNA, specifically the small RNA is a siRNA, tasiRNA, snRNA, miRNA, snoRNA, piRNA and tRNA and any microRNA precursors thereof.

According to a specific embodiment of the invention, the core nucleotide sequence comprises from 8 to 25 nucleotides, preferably from 10 to 20 nucleotides, preferably from 12 to 18 nucleotides, preferably 8, 9, 10, 11, 12, 13, 14, 15, 16, 17, 18, 19, 20, 21, 22, 23, 24,25 nucleotides. The cores sequences of the different subsets are specifically differing in 1, 2, 3, 4, 5, 6, 7, 8, 9, 10, 11, 12, 13, 14, 15, 16, 17, 18 19 20, 21, 22, 23, 24 or 25 nucleotides.

Specifically, the core sequences comprise 3 or more mismatching nucleobases to the target sequence.

Specifically, the core sequence contains 100% mismatches compared to the respective target sequence. E.g., if the core sequence has a length of 8 nucleotides, the core nucleotide sequence comprises 8, specifically 7, 5, 4, 3, or 2 mismatching nucleobases compared to the target sequence. As a further example, a core oligonucleotide of 25 nucleotides shall comprise 25 24, 23, 22, 21, 20, 19, 18, 17, 16, 15, 14, 13, 12, 11, 10, 9, 8, 7, 5, 4, 3 or 2 mismatching nucleobases compared to the target sequence.

Also encompassed herein is a core nucleotide sequence which is different from, i.e. is not complementary to a gene or the genome of an organism of interest. Specifically, the core nucleotide sequence contains at least two mismatching nucleobases compared to the target sequence, more specifically the core sequence contains 100%, 97.5%, 95%, 90%, 85%, 80%, 75%, 70%, 65%, 60%, 55%, 50%, or more mismatches compared to the gene or the genome of an organism of interest.

According to a specific embodiment, the sequence of randomized nucleotides comprises 3 to 7 nucleotides, preferably 3 to 5 nucleotides, preferably 4 nucleotides. Alternatively, it comprises 3, 4, 5, 6, 7 or more nucleotides.

The set of the invention can comprise 2, 3, 4, 5, 6, 7, 8, 9, 10, 11, 12, 13, 14, 15, 16, 17, 18, 19, 20, 21, 22, 23, 24 or more subsets, specifically in an amount from 0.001 to 50000 amol, specifically from 0.01 to 25000 amol, specifically from 1 to 10000 amol, specifically from 10 to 5000 amol per subset.

The target sequence used according to the invention can be any sequence of interest, specifically it can be a genome of an organism, a sequence originating from virus, bacteria, animals, plants, specifically it can be a transcriptome, an RNA, a small RNA, a dynamic small RNA population.

The sets of the invention can further be used in methods for determining the amount of one or more target sequences in a sample, specifically in a cell, a tissue or an organ sample.

In another aspect, provided herein is a method for generating a library of nucleic acids, specifically comprising small RNAs or sRNA mimics, for use in sequencing methods, wherein one or more sets of the invention are mixed with target nucleic acid molecules. Said libraries of course can be further amplified.

In another aspect, provided herein is a method for determining reference values in nucleotide sequencing, comprising the steps of adding one or more sets according to the invention to a mixture of target sequences, thereby generating a library of nucleic acid molecules, ligating adapters to the nucleic acid molecules, optionally amplifying said library, performing a nucleotide sequencing method, and determining the amount of nucleic acid molecules of each subset as reference value.

In another aspect, provided herein is a method for determining the absolute amount of a target sequence, wherein the amount of the target sequences is compared with the reference values.

The invention further encompasses a method for determining the number of nucleic acid molecules in a sample, comprising the steps of
a. adding a set of the invention to the sample to get a mixture of nucleic acid molecules, thereby generating a library of nucleic acid molecules,
b. ligating and optionally amplifying said library,
c. performing Next Generation Sequencing of said library resulting in RNA sequence reads from said nucleic acid molecules,
d. determining the number of reads from the set and from the sample.

Specifically, the above method can be used for absolute quantification of small RNA molecules in a sample, more specifically for absolute normalizing sRNA sequence data from different sources.

As an alternative embodiment, the invention provides for an oligonucleotide described by the general formula:

p-(N)ₘ(x)ₙ(N)ₘ-2'-O-methyl,

wherein
N is a random nucleotide of any of A, U, G, C or A, T, G, C;
X is the core nucleotide sequence comprising any of A, U, G, C and containing two or more mismatches compared to a target sequence,
m is 3, 4 or 5, and
n is an integer in the range of 8 to 67.

In a specific embodiment, the core sequence is not complementary to the target sequence.

In another aspect, provided herein is a method for preparing spike-ins for sequencing, wherein at least two different oligonucleotides, specifically a plurality of oligonucleotides as described above are used, specifically containing all possible nucleotide combinations of A, U, C, G

### Figures

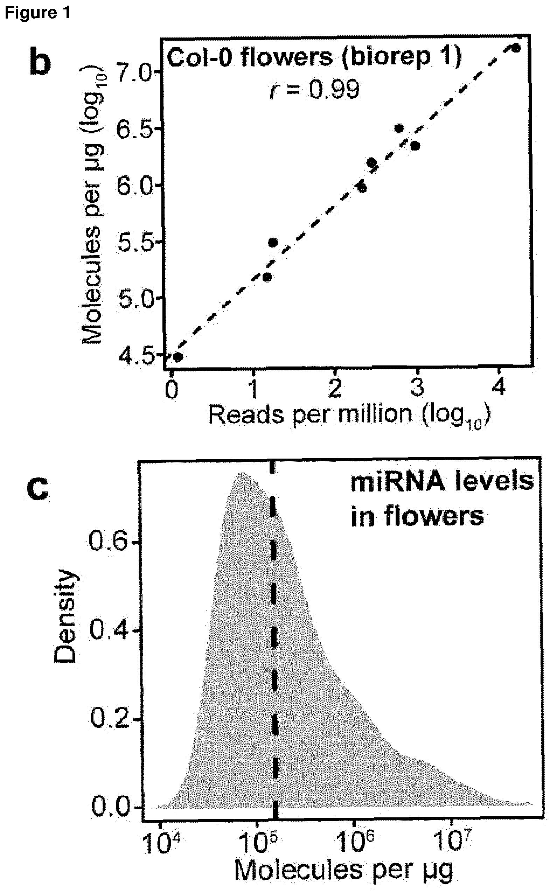

**Figure 1****:** Small RNA spike-in design and use as a tool to examine cloning biases and estimate absolute small RNA levels. (a) Scatter plot of relative small RNA spike-in levels (reads per million genome-matching reads) compared to absolute small RNA levels (molecules per µg of total RNA) in Col-0 flowers (biological replicate 1). Pearson's *r* value is indicated, as well as a dashed line that represents a linear model derived from the plotted values. (b) Density plot of individual miRNA family molecules per µg of total floral bud RNA. Vertical dashed line indicates the median number of molecules per miRNA family.

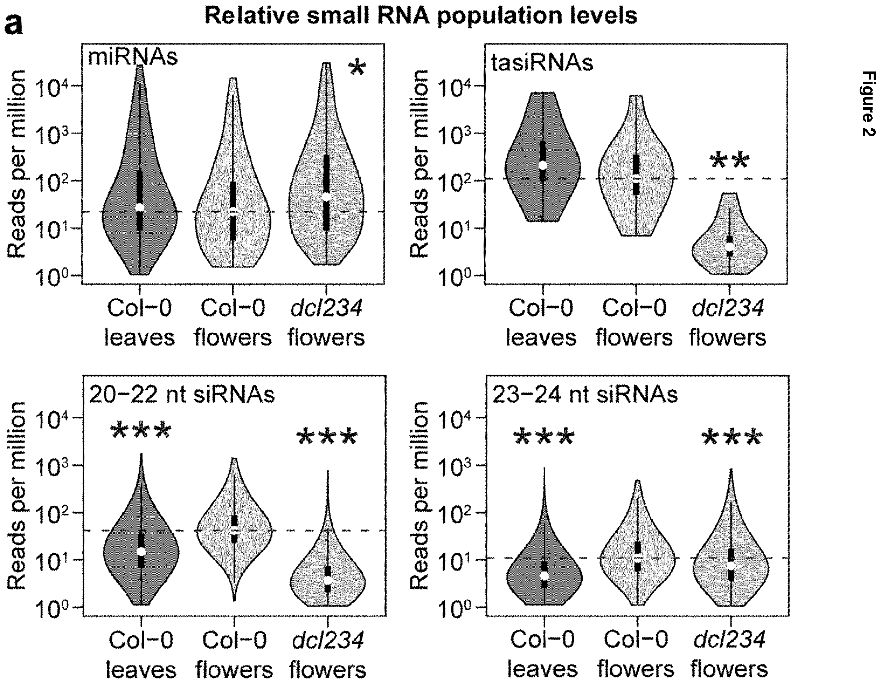

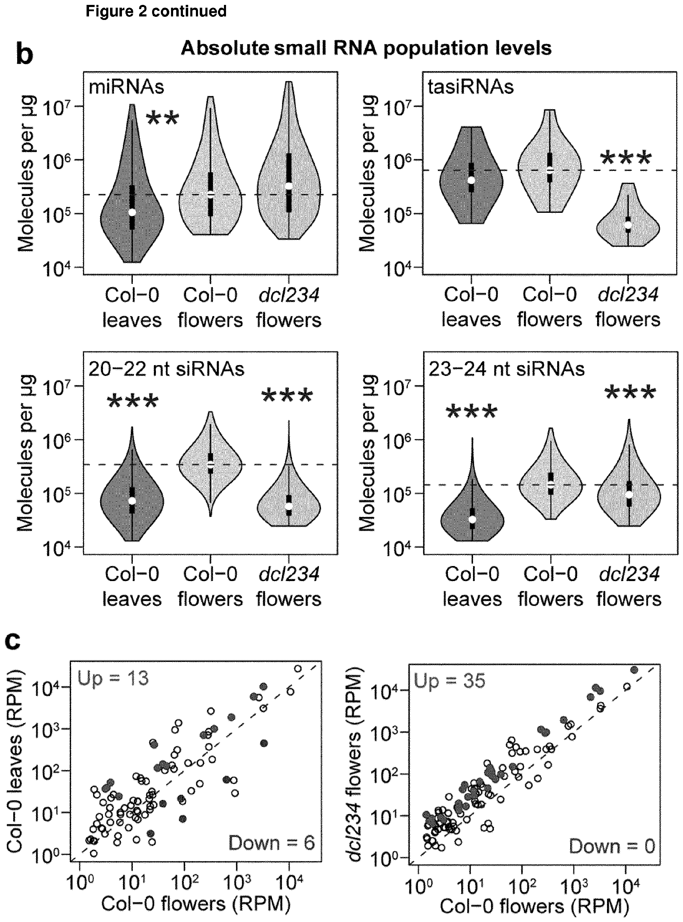

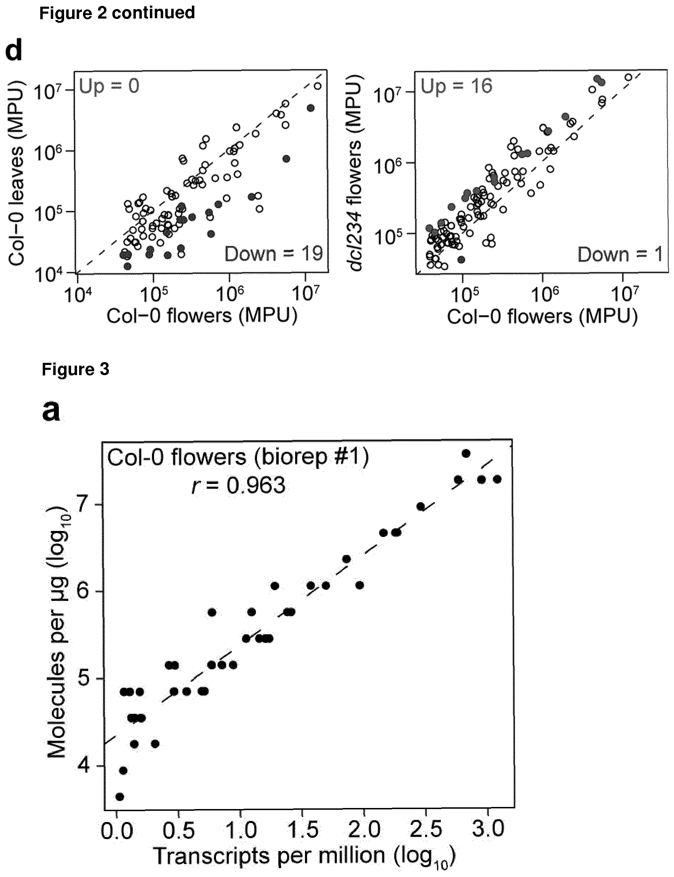

**Figure 2****:** Small RNA spike-ins enable accurate comparisons of small RNA levels. (a and b) Violin plots of individual miRNAs, tasiRNAs and siRNA family levels in either relative (a) or absolute (b) units. P-values were based on two-sample Kolmogorov-Smirnov tests. *P* < 0.05, *P <* 0.01 and *P <* 0.001 are indicated by *, ** and ***, respectively. Violin plots are composed of 93 miRNA, 14 tasiRNA, 6,361 20-22 nt siRNA and 5,952 23-24 nt siRNA families with ≥1 RPM in each sample. The 25^{th} and 75^{th} percentiles are indicated by the bottom and top of the vertical black bar in the violin plots, while the medians are indicated by white points within the vertical black bars. The top and bottom whiskers extend from the vertical black bar to the most extreme values within 1.5 times the interquartile range. The widths of the violins are proportional to the sample densities. (c and d) Scatter plots of miRNA family levels in Col-0 flowers vs. Col-0 leaves (left) and Col-0 flowers vs. *dcl234* leaves (right) in either relative RPM (c) or absolute MPU (d) terms. Full black points indicate miRNA families with significantly increased and significantly decreased levels, respectively. Significantly different miRNA levels were defined as having ≥2-fold different and a false discovery rate adjusted p-value < 0.05 based on a two-sample Student's t-test.

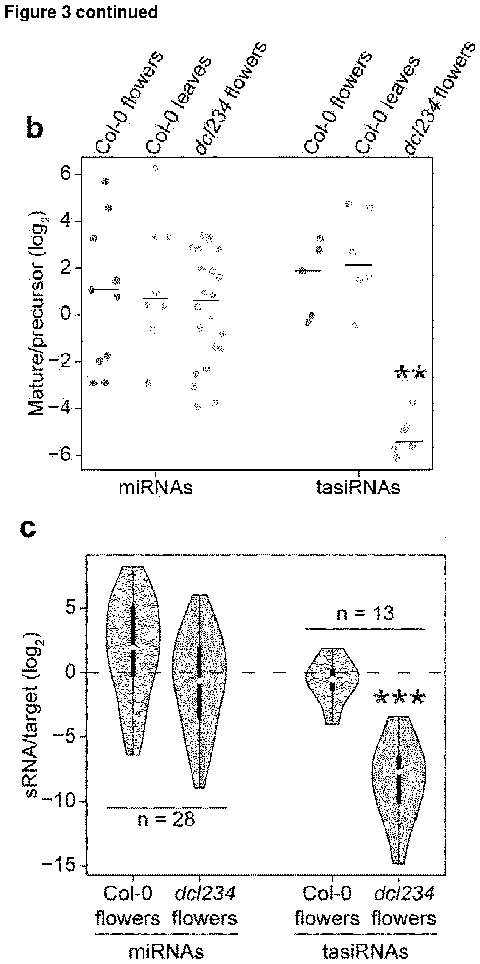

**Figure 3****:** Combined use of small RNA and poly(A) RNA spike-ins allow direct comparisons of small RNA-Seq and mRNA-Seq data. (a) Scatter plot of relative (transcripts per million) and absolute (molecules per µg total RNA) ERCC poly(A) spike-in (LifeTech) levels. Pearson's *r* value is indicated, as well as a dashed line that represents a linear model derived from the plotted values. (b) One-dimensional scatter plots of miRNA/miRNA precursor and tasiRNA/tasiRNA precursor levels in Col-0 leaves, Col-0 flowers and *dcl234* flowers. (c) Violin plots of miRNA/target and tasiRNA/target levels in Col-0 leaves, Col-0 flowers and *dcl234* flowers. Violin plots are as described in the Fig. 2 legend. *P <* 0.01 and *P <* 0.001 are indicated by ** and ***, respectively, and were calculated with two-sample Kolmogorov-Smirnov tests. The number (n) of miRNA:target and tasiRNA:target interactions examined is indicated.

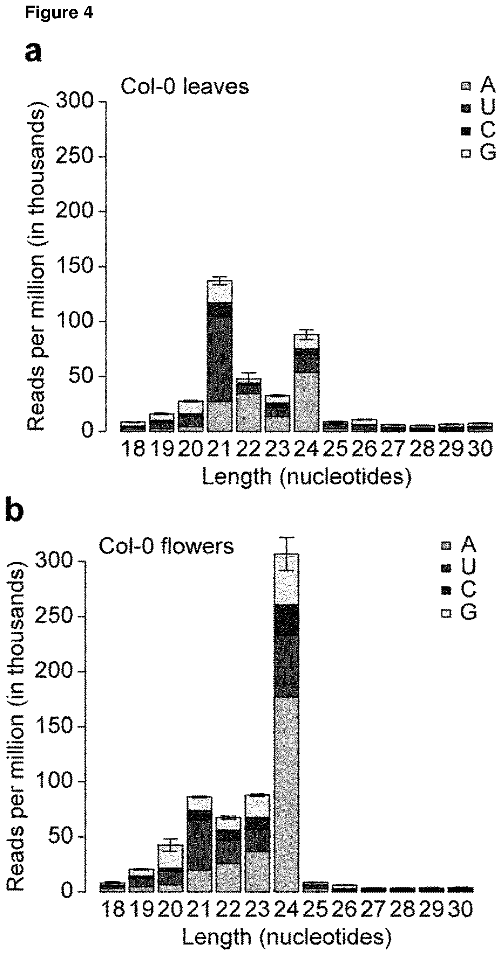

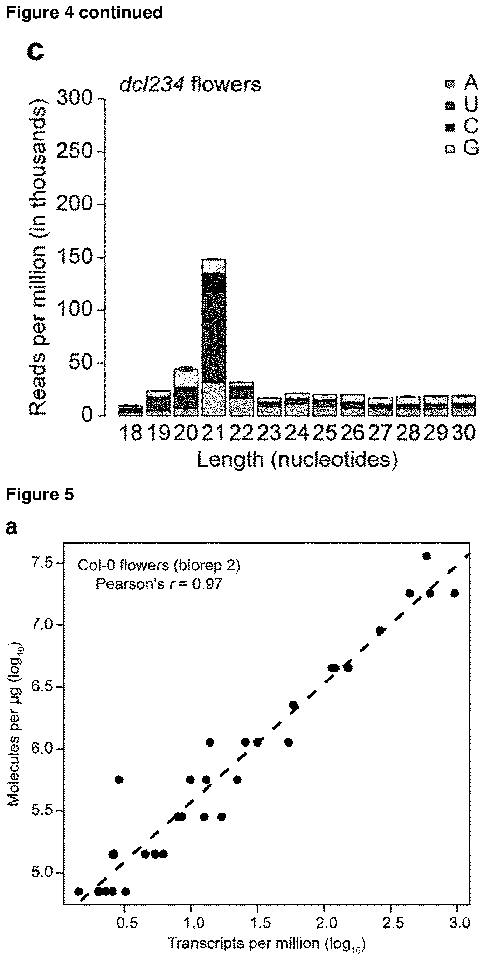

**Figure 4****:** Length distributions of small RNA populations. Stacked bar plots of RPMs for different size classes of sRNAs in Col-0 leaves (a), Col-0 flowers (b) and *dcl234* flowers (c). Colors indicate the proportion of sRNA-Seqs that begin with the indicated nucleotides.

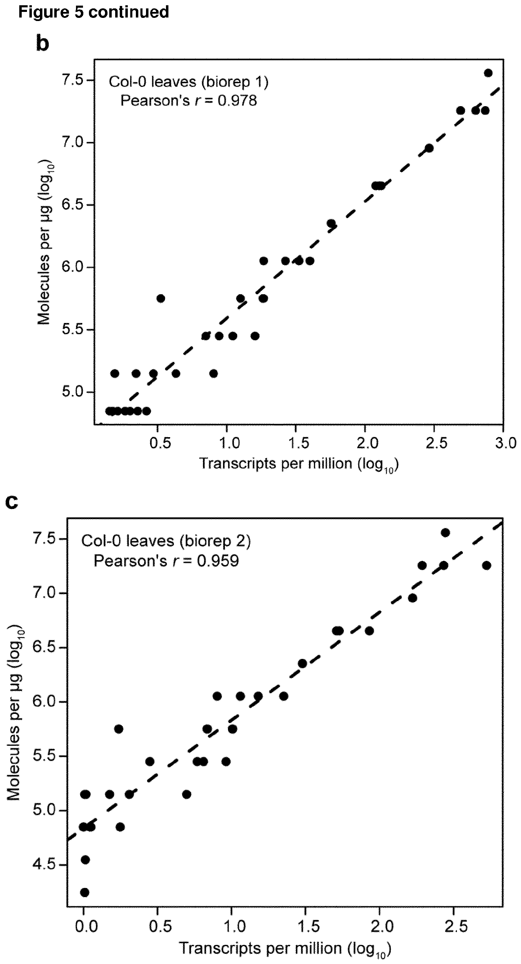

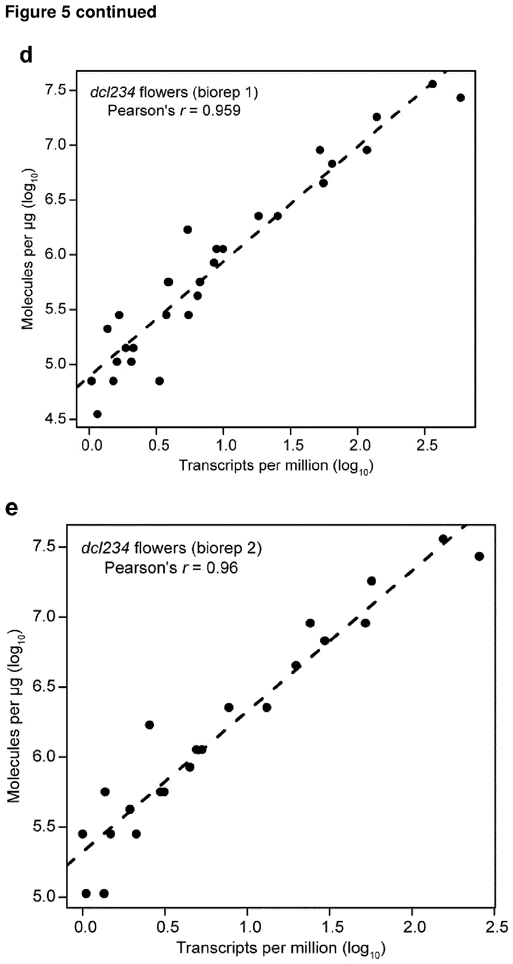

**Figure 5****:** Standard curves used to estimate the number of mRNA molecules per µg of total RNA. Scatter plots of relative (transcripts per million) and absolute (molecules per µg total RNA) ERCC poly(A) spike-in levels for mRNA-Seq libraries generated from wild-type (Col-0) flowers (biological replicate 1) (a), Col-0 leaves (biological replicates 1 and 2) (b and c) and *dcl234* flowers (biological replicates 1 and 2) (d and e). Pearson's *r* values are indicated, as well as dashed lines that represent linear models derived from the plotted values.

### Detailed description

Specific terms as used throughout the specification have the following meaning.

The term "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/- 10% of the given value.

A "molecule" as used herein refers to a single entity or particle made up of atoms by covalent bonds. The molecule can comprise different parts, which are made up of one single class of substances or a combination thereof. Classes of substances are, for example, but not limited to, single-stranded DNA (ssDNA), single-stranded RNA (ssRNA), including DNA and/or RNA with chemical modifications, a peptide or other organic compound. The molecule may be of varying length, size or molecular weight, and can have any activity known and/or desired by the skilled person.

As used herein, the terms "nucleic acid", "polynucleotide", and "polynucleic acid" can be used interchangeably and refer to a polymeric form of nucleotides of any length, either ribonucleotides, deoxyribonucleotides, chemically modified forms or analogs of nucleotides, and combinations of the foregoing. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g. altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g. rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudourdine, dihydrouridine, queuosine, and wyosine. A single-stranded nucleic acid can also be one strand nucleic acid of a denatured double-stranded DNA. Alternatively, it can be a single-stranded nucleic acid not derived from any double-stranded DNA. In one aspect, the template nucleic acid is RNA. In another aspect, the template is DNA. The term includes sense and anti-sense strands of RNA, cDNA, genomic DNA, synthetic forms and mixed polymers thereof. The term also includes any topological conformation, including single-stranded (ss) and double stranded (ds) conformations.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines.

Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. Within nucleic acids, the phosphate groups can commonly be referred to as forming the internucleoside backbone of the nucleic acid. The linkage or backbone of the nucleic acid can be a 3' to 5' phosphodiester linkage. A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkylphosphotriesters, methyl and other alkyl phosphonates such as 3'-alkylene phosphonates, 5'-alkylene phosphonates, chiral phosphonates, phosphinates, phosphoramidates including 3'-amino phosphoramidate and aminoalkylphosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can also include nucleobase (often referred to simply as "base") modifications or substitutions. As used herein, nucleobases include the purine bases, (e.g. adenine (A) and guanine (G)), and the pyrimidine bases, (e.g. thymine (T), cytosine (C) and uracil (U)).

RNA mimics are chemically modified double-stranded RNAs that mimic native endogenous RNAs. miRNA mimics simulate naturally occurring mature microRNAs, e.g. they contain non-natural or artificial double stranded miRNA-like RNA fragments.

Dynamic small RNA refers to small RNA which is differently expressed in different development stages, tissues, cell types and cellular compartments.

The term "nucleic acid molecule", as used herein, is intended to include RNA molecules, DNA molecules, cDNA molecules, oligonucleotides, polynucleotides and analogs or derivatives thereof. The term includes synthetic nucleic acid molecules, such as chemically synthesized, chemically modified or recombinantly produced nucleic acid molecules. Suitable nucleic acid molecules are RNA, including any small RNA but not limited thereto, for example mRNA, siRNA, tasiRNA, snRNA, miRNA, snoRNA, piRNA, and tRNA.

The nucleic acid molecules of the invention specifically are single stranded (ss). Specifically, the single stranded nucleic acid molecule is an RNA molecule. The single stranded nucleic acid molecule can be the sense strand or the antisense strand.

The term "oligonucleotide" as used herein denotes a single stranded multimer of nucleotides of from about 2 to 200 nucleotides, specifically 2-100, specifically 2 to 75 nucleotides in length. Oligonucleotides may be synthetic or may be made enzymatically. Oligonucleotides may contain ribonucleotide monomers (i.e., may be oligoribonucleotides) or deoxyribonucleotide monomers, or both ribonucleotide monomers and deoxyribonucleotide monomers. An oligonucleotide may have, but is not limited to, a length of 2-75, 4-50, 5-40, 10-35, 15 - 30, 17 - 25, 20, 21, 22, 23, 24, 25, 25, 27, 28, 29, 30 nucleotides.

Small RNA mimics can also be used in the inventive oligonucleotide sets and can be small RNAs with different 5' and 3' modifications, containing a 5' monophosphate, diphosphate or triphosphate, containing a 3'OH or O-methyl or 2'O-methyl. Small RNA mimics contain less than 200 nucleotides, specifically less than 100 nucleotides, specifically less than 75 nucleotides, specifically less than 50 nucleotides, specifically between 8 and 50 nucleotides, more specifically between 20 and 35 nucleotides. Artificial RNA oligonucleotides mimicking plant RNAs can be for example, oligonucleotides that contain 5'monophosphate and 3'OH.

An oligonucleotide of the present invention may be characterized following general formula:

p-(N)ₘ(X)(N)ₙ2*'*-O-methyl

, wherein
p is a phosphate,
N is a random nucleotide of any of A, U, G, C or A, T, G, C,
X is a core sequence which sequence contains two or more nucleobase mismatches compared to a target sequence having a length of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67 comprising any of A, U, G, C; or A, T, G, C; and
m, n are 3, 4 or 5.

Specifically, the number of mismatches is determined in a way to result in a core sequence that is not complementary to the target sequence.

The term "chemically modified nucleotide" refers to nucleotides, which differ in their chemical structure from conventional nucleotides, having modifications in the chemical structure of the base, sugar and/or phosphate. Nucleotides can be modified at any position of their structure. Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine (1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g. 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

The term "adapter" sequence as used herein refers to a short, chemically synthesized, double stranded DNA molecule which is used to link the ends of other DNA molecules, specifically for fishing an unknown DNA sequence. As an embodiment, short sequences are attached to the ends of fragments of the target sequences and to the oligonucleotides comprised in the inventive sets. Adapter sequences are useful for sequencing random fragments of DNA. The addition of short nucleotide sequences allow any DNA fragment to bind to a flow cell for next generation sequencing. Examples for methods of library construction using adapter sequences is described in Head S. et al., 2015 (Biotechniques, 56(2), 61-passim). Exemplarily, adapters are used as described in the user manual for NEBNEXT Small RNA Library Prep set for Illumina (Multiplex Compatible, https://www.neb.eom/∼/media/Catalog/All-Products/ 7D0645075EAA4A07843194C69EB391A7/Datacards%20or%20Manuals/manualE733 0.pdf), specifically the 3'adapters are of the sequence: 5'-rAppAGATCGGAAGAGCACACGTCT-NH₂-3' (SEQ ID NO 9) and the 5'adapters are of the sequence 5'- rGrUrUrCrArGrArGrUrUrCrUrArCrArGrUrCrCrGrArCrGrArUrC-3' (SEQ ID NO 10). As an alternative, any other adaptor may be used which can be provided by the skilled artisan.

The term "complementarity" or "complementary" as used herein refers to the formation or existence of hydrogen bond(s) between one nucleic acid sequence and another nucleic acid sequence by either traditional Watson-Crick or other non-traditional types of bonding as described herein. Perfect or 100% complementary means that all the contiguous residues (nucleobases) of a nucleic acid sequence will hydrogen bond with the same number of contiguous residues in a second, complementary, nucleic acid sequence. According to a specific aspect, the contiguous residues of the first nucleic acid sequence are identical with the contiguous nucleic acids of the second sequence. "Non-complementarity" includes various mismatches or non-based paired nucleotides (e.g., 2, 3, 4, 5, 6, 7, 8, 9, 10, 11, 12, 13, 14, 15, 16, 17, 18, 19, 20 or more mismatches, non-nucleotide linkers, or non-base paired nucleotides) within the nucleic acid molecule, which can result in bulges, loops, or overhangs between the two nucleic acid sequences. Such non complementarity can be represented by a % complementarity that is determined by the number of non-base paired nucleotides, i.e., about 50%, 60%, 70%, 80%, 90%, 91%, 92%, 93%, 94%, 95%, 96%, 97%, 98%, 99% etc. within the total number of nucleotides involved. For reasons of completeness, also reverse complementarity is encompassed herein.

Herein, the term "not complementary" of the core sequences can be represented by a % complementarity that is determined by the number of non-base paired nucleotides, i.e., refers to at least 50%, specifically at least 55%, 60%, 65%, 70%, 75%, 80%, 85%, 90%, 91%, 92%, 93%, 94%, 95%, 96%, 97%, 98%, 99% non-base paired nucleotides or at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence mismatches of the core sequence with reference to a target sequence of interest.

The term "hydrogen bond" as used herein, refers to a form of association between an electronegative atom and a hydrogen atom attached to a second atom exceeding the electronegativity of carbon. The electronegative-atom having a free electron pair to share with the hydrogen atom is the so-called hydrogen bond acceptor, and may be nitrogen, oxygen, sulfur or fluorine. The hydrogen atom bound to the electronegative atom is generally referred to as a hydrogen bond donor. The terms electronegative and electropositive as used herein will be readily understood by the person skilled in the art to mean the tendency of an atom to attract the pair of electrons in a covalent bond so as to lead to an unsymmetrical distribution of electrons and hence the formation of a dipole moment. The hydrogen bond is stronger than a van der Waals interaction, but weaker than covalent or ionic bonds.

The term "hybridize" or "anneal" refers to the ability of nucleic acid strands to come together under specified hybridization conditions in a parallel or preferably antiparallel orientation. The nucleic acid strands interact via hydrogen bonding between bases on opposing strands and form a stable or quasi-stable double-stranded helical structure or may result in the formation of a triplex, or other higher-ordered structure. Although hydrogen bonds typically form between adenine and thymine or uracil (A and T or U) or cytosine and guanine (C and G), other base pairs may be formed (e.g., Adams et al., The Biochemistry of the Nucleic Acids, 11 th ed., 1992). The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of co-solvents, lowering the salt concentration, and the like.

As will be appreciated by persons skilled in the art, stringent conditions are sequence-dependent and are different in different circumstances. For example, longer fragments may require higher hybridization temperatures for specific hybridization than short fragments. Because other factors, such as base composition and length of the complementary strands, presence of organic solvents and ions, and the extent of base mismatching, may affect the stringency of hybridization, the combination of parameters can be more important than the absolute measure of any one parameter alone. In some embodiments, hybridization can be made to occur under high stringency conditions, such as high temperatures or 0.1 X SCC. Examples of high stringent conditions are known in the art; see e.g., Sambrook et al., Molecular Cloning: A Laboratory Manual, 2d Edition, 1989, and Short Protocols in Molecular Biology, ed. Ausubel et al. In general, increasing the temperature at which the hybridization is performed increases the stringency. As such, the hybridization reactions described herein can be performed at a different temperature depending on the desired stringency of hybridization. Hybridization temperatures can be as low as or even lower than 5 °C, but are typically greater than 22 °C, and more typically greater than about 30°C, and even more typically in excess of 37 °C. In other embodiments, the stringency of the hybridization can further be altered by the addition or removal of components of the buffered solution. In some embodiments, hybridization is permitted under medium stringency conditions. In other embodiments, hybridization is permitted under low stringency conditions. In some embodiments, two or more mismatches are present between the hybridized molecules or hybridized portions of molecules.

The term "identity" or "identical" in the context of two or more nucleic acid sequences, refer to two or more sequences or subsequences that have a specified percentage of nucleotides that are the same, when compared and aligned for maximum correspondence, as measured using one of the sequence comparison algorithms described below (e.g., BLAST, BLAST-2, BLASTN, ALIGN, ALIGN-2, Megalign (DNASTAR) or the needle pairwise sequence alignment (EMBOSS software) or other algorithms available to persons of skill) or by visual inspection. Depending on the application, the percent "identity" can exist over a region of the sequence being compared, e.g., exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

Not identical means that there is less than 100% nucleic acid sequence identity, i.e. at least 2, 3, 4, 5, or more nucleotides from a first sequence differ from the nucleotides from a second sequence. Alternatively, sequence identity is 80%, 75%, 70%, 65%, 60%, 55%, 50%, 45%, 40%, 35%, 30%, 25%, 20%, 15%, 10%, 5% or less.

As used herein, the term "gene" relates to genetic nucleotides with a sequence that is transcribed to form one or more transcripts. Specifically, gene refers to a locus (or region) of DNA which is made up of nucleotides that are transcribed (DNA) to RNA in vitro or in vivo when operably linked to appropriate regulatory sequences. The gene can include regulatory regions preceding and following the coding region, e.g. 5' untranslated (5'UTR) or "leader" sequences and 3' UTR or "trailer" sequences, as well as intervening sequences (introns) between individual coding segments (exons). Specifically, the gene encodes a protein (e.g. mRNA), but also stipulated are non protein-coding transcripts, such as regulatory or catalytic RNA, including microRNA, snoRNA or rRNA and their precursors, pre-microRNA and pre-rRNA.

The terms "gene of interest" or "genome of interest" refers to genes or parts or functional fragments thereof or genomes originating from a sample from any organism or any synthetic genes or modified genes derived from any organism, such as, but not limited to virus, bacteria, fungi, plants, animals etc.

The term "transcriptome" refers to the total set of transcripts, such as messenger RNA (mRNA) molecules, small interfering RNA (siRNA) molecules, transfer RNA (tRNA) molecules, ribosomal RNA (rRNA) molecules, in a given sample or organism, or to the specific subset of transcripts present in a particular cell type. Unlike the genome, which is roughly fixed for a given cell line (excluding mutations), the transcriptome can vary with external environmental conditions. Because it includes all transcripts in the cell, the transcriptome reflects the genes that are actively expressed at any given time, with the exception of mRNA degradation phenomena such as transcriptional attenuation. In some embodiments, transcriptome not only refers to the species of transcripts, such as mRNA species, but also the amount of each species in the sample. In some embodiments, a transcriptome includes each mRNA molecule in the sample, such as all the mRNA molecules in a single cell.
"Target sequence" refers to a nucleotide sequence of interest, including a nucleic acid molecule of interest, a gene of interest or genome of interest or any parts, derivatives or fragments thereof, such as, but not limited to a nucleotide sequence originating or derived from virus, bacteria, fungi, animals, plants. Target sequences may also be transcriptomes, RNA sequences or fragments thereof, specifically RNA, total RNA, size-selected total RNA, small RNA and dynamic small RNA from any source. Specifically, the target sequence is a non-coding or coding sequence or a combination thereof. Specifically, the target sequence encodes or regulates a metabolic or biosynthetic pathway or a part of such pathway, e.g. respective enzymes or regulators of such pathway. Specifically, the target sequence encodes a single biomolecule such as an enzyme, or a ligand-binding protein, an antibody, a structural protein, or a protein with other function, a ribozyme, a riboswitch, a regulatory RNA, or any other RNA molecule, or a group of biomolecules that form a cellular pathway, a regulatory network, a metabolic pathway, or a cellular subsystem, or a part of any of the foregoing.

In various embodiments, the nucleic acid is amplified. Any amplification method known in the art may be used. Examples of amplification techniques that can be used include, but are not limited to, PCR, quantitative PCR, quantitative fluorescent PCR (QF-PCR), multiplex fluorescent PCR (MF-PCR), real time PCR (RT-PCR), single cell PCR, restriction fragment length polymorphism PCR (PCR- RFLP), hot start PCR, nested PCR, in situ polony PCR, in situ rolling circle amplification (RCA), bridge PCR, picotiter PCR, and emulsion PCR. Other suitable amplification methods include the ligase chain reaction (LCR), transcription amplification, self-sustained sequence replication, selective amplification of target polynucleotide sequences, consensus sequence primed polymerase chain reaction (CP -PCR), arbitrarily primed polymerase chain reaction (AP-PCR), degenerate oligonucleotide-primed PCR (DOP-PCR), and nucleic acid based sequence amplification (NABSA).

The term "sequencing" includes any method of determining the sequence of a nucleic acid. Such methods include Maxam-Gilbert sequencing, Chain-termination methods, Shot gun sequencing, PCR sequencing, Bridge PCR, massively parallel signature sequencing (MPSS), Polony sequencing, pyrosequencing, Illumina (Solexa) sequencing, SOLiD sequencing, Ion semiconductor sequencing, DNA nanoball sequencing, Heliscope single molecule sequencing, Single molecule real time (SMRT) sequencing, Nanopore DNA sequencing, sequencing by hybridization, sequencing with mass spectrometry, microfluidic Sanger sequencing, microscopy-based techniques, RNAP sequencing, (in vitro virus) high- throughput sequencing (HTS).

The term "next-generation sequencing (NGS)" refers to the so-called parallelized sequencing by synthesis or sequencing by ligation platforms currently employed by Illumina, Life Technologies, and Roche etc. Next-generation sequencing methods may also include nanopore sequencing methods or electronic-detection based methods such as Ion Torrent technology commercialized by Life Technologies.

Specifically, NGS refers to a high- throughput sequencing technology that performs thousands or millions of sequencing reactions in parallel. Although the different NGS platforms use varying assay chemistries, they all generate sequence data from a large number of sequencing reactions running simultaneously on a large number of templates. Typically, the sequence data is collected using a scanner, and then assembled and analyzed bioinformatically. Thus, the sequencing reactions are performed, read, assembled, and analyzed in parallel; see, e.g. Behjati S and Tarpey, P., 2013 (Arch.Di.Child Educ Pract Ed 2013, 98, 236-238); Head S. et al., 2015 (Biotechniques, 56(2), 61-passim).

Some NGS methods require template amplification and some do not. Amplification requiring methods include pyrosequencing (e.g., U.S. Pat. No.6,258,568; commercialized by Roche); the Solexa/lllumina platform (e.g., U.S. Pat. Nos. 6,833,246, 7,115,400, and 6,969,488); and the Supported Oligonucleotide Ligation and Detection (SOLiD) platform (Applied Biosystems; e.g., U.S. Pat. Nos. 5,912,148 and 6,130,073). Methods that do not require amplification, e.g., single-molecule sequencing methods, include nanopore sequencing, HeliScope (U.S. Pat. Nos. 7,169,560; 7,282,337; 7,482,120; 7,501,245; 6,818,395; 6,911,345; and 7,501,245); real-time sequencing by synthesis (see, e.g., U.S. Pat. No. 7,329,492); single molecule real time (SMRT) DNA sequencing methods using zero-mode waveguides (ZMWs); and other methods, including those described in U.S. Pat. Nos. 7,170,050; 7,302,146; 7,313,308; and 7,476,503, US 20130274147; US20140038831; and Metzker, Nat Rev Genet 11(1): 31-46 (2010). Alternatively, hybridization-based sequence methods or other high-throughput methods can also be used, e.g., microarray analysis, NANOSTRING, ILLUMINA, or other sequencing platforms.

The term "subset" refers to single stranded nucleic acid molecules having identical core sequences and different randomized nucleotides as described herein. The nucleic acid molecules of each set may also have the same or different modifications at the 3'and/or 5'sites. Specifically, the nucleic acid molecules of one set contain identical 3'and 5'modifications. A subset also refers to a specific amount or number of nucleic acid molecules having identical core sequences.

Each subset can comprise an amount of nucleic acid molecules from about 0.001 to 50000 amol, 1 to 10000 amol, from about 1 to 8000 amol, from 10 to 5000 amol. Specifically the subsets can comprise an amount of nucleic acid molecules of about 0.001, 0.01, 0.1, 1, 10, 25, 30, 40, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, 9500, 10000 amol.

The term "set" refers to two or more subsets of nucleic acid molecules, which can contain the same or different amounts of oligonucleotides. The number of subsets of a set can be 2, 3, 4, 5, 6, 7, 8, 9, 10 or more and is not limited. One or more sets can be used for one sequencing.

A "plurality" of nucleic acid molecules as used herein contains at least 2 members. In certain cases, a plurality may have at least 2, at least 5, at least 8, at least 10, at least 100, at least 1,000, at least 10,000, at least 100,000, at least 10⁶, at least 10⁷, at least 10⁸ or at least 10⁹ or more members. Specifically, the term plurality refers to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more members.

The inventive sets can be used as spike-in probes which are RNA transcripts used to calibrate measurements in an experiment, specifically in a sequencing method like NGS. Spike-ins are external references at constant levels across a sample set. Spike-ins are synthetic oligonucleotides, RNA or DNA sequences which can facilitate objective normalization between different data sets. Optimized spike-ins of the invention are designed so that these spike-ins do not to hybridize or show low hybridization with a specific sequence or region of the target sequence. Known amounts of the sets or oligonucleotides of the present invention are mixed with the sequence of interest during preparation. Spike-ins containing different amounts of subsets can be used as spike-in cocktails. Beneficially, the spike-ins are added, specifically as reference sets, early during sample preparation, specifically before adapter ligation or detection or quantification method, so that this reference set is present during all or most sample preparation steps. After sequencing, the read count of the spike in sequences is used to determine the direct correspondence between read count and target sequence. Thus for quantitation signal intensities (read counts) representing the expression levels of the experimental gene, exon, or target sequence are related to standards which contain known quantities and were defined as absolute references. In developmental studies, this technique is used to determine transcription kinetics. By using the inventive sets of the invention, an appropriate control can be provided which avoids any errors due to the use of internal RNA standards as these genes are seldom expressed at the same level at all stages of development.

"Mismatch" refers to a change of a nucleobase compared to a template nucleotide sequence which specifically is a target nucleotide sequence. Specifically, the core nucleotide sequence is a semi-randomized sequence. Specifically, the nucleobase mismatches or the point mutations (in particular those introduced into the core nucleotide sequence) are selected from the group consisting of at least two nucleobase or nucleotide substitutions, insertions or deletions, codon substitutions, or combinations thereof, specifically the nucleobase mismatches can be single, double or triple, consecutive mismatches.

The term "randomization" or "randomized sequence" shall refer to specific nucleotide sequence modifications in a predetermined region. Randomization results in a repertoire of nucleic acids. Randomized is a term used to describe a segment of a nucleic acid having, in principle any possible sequence over a given length. Randomized sequences can be of various lengths, as desired, ranging from about 2 to more than 100 nucleotides. The chemical or enzymatic reactions by which random sequence segments are made may not yield mathematically random sequences due unknown biases or nucleotide preferences that may exist. In the techniques presently known, for example sequential chemical synthesis, large deviations are not known to occur. For short segments of 20 nucleotides or less, any minor bias that might exist would have negligible consequences. The longer the sequence of a single synthesis, the greater is the effect of any bias.

Random nucleic acids can be obtained in a number of ways. For example, full or partial sequence randomization can be readily achieved by direct chemical synthesis of the nucleic acid (or portions thereof) or by synthesis of a template from which the nucleic acid (or portions thereof) can be prepared by use of appropriate enzymes. End addition, catalyzed by terminal transferase in the presence of non-limiting concentrations of all four nucleotide triphosphates, can add a randomized sequence to a segment.

Sequence variability in nucleic acids can also be achieved by employing size-selected fragments of partially digested (or otherwise cleaved) preparations of large, natural nucleic acids, such as genomic DNA preparations or cellular RNA preparations. A randomized sequence of 30 nucleotides will contain calculated 10¹⁸ different candidate sequences.

The random sequence of the present invention is represented as "N". N comprises a random oligonucleotide sequence of 2, 3, 4, 5, 6, 7, 8 or more different nucleotides. Random sequences of ≤6, specifically ≤5, specifically ≤4 are preferred. The number of possible nucleotide sequences of length X is 4^{X}, thus a random nucleotide segment of even a short length may encode many possible unique nucleotide sequences. N can be any of the nucleotides A, C, G, U, T.

Random sequences allow each synthetic template oligonucleotide to be quantitated exactly. Upon amplification of a pool of synthetic template oligonucleotides, each unique random nucleotide sequence observed in the sequencing output represents a single molecule of input material. Thus, the input number of synthetic template oligonucleotides added to the amplification reaction can be determined by counting the number of unique random nucleotide sequences. Furthermore, the input number of synthetic template oligonucleotides is associated with a particular barcode.

Semi-random sequences may be generated by different methods. Exemplary, a matrix consisting of the proportions of base identities for miRNA positions of the core sequence (counting from the 5' end of the miRNA) of the most highly abundant miRNAs can be generated, and used to semi-randomly select several hundred and up to 1000 or more sequences of a specific length, e.g. having a length of 8-21, specifically of 13 nucleotides. Sequences that do not perfectly align to the genome of interest may be considered further and all possible 4-base combinations can be added to both the 5' and 3' ends as random sequences making the total sequences per set. The minimum free energies of all synthesized RNAs can be determined using methods known in the art. These minimum free energy distributions can be examined and sets of RNA sequences with distributions similar to annotated miRNAs can be selected and synthesized.

According to an embodiment, the core sequence contains nucleotide sequences generated by semi-randomization.

Libraries of semi-random sequences may also be used consisting of a pool of variant oligonucleotides each of which differs by a single nucleotide alteration.

The present invention provides a method and a set of oligonucleotide subsets for absolute normalization of sequence data, specifically of small RNA sequence data, the set comprising at least two subsets of single stranded nucleic acid molecules, each nucleic acid molecule comprising from the 5'to 3'direction:
a) a 5'phosphate,
b) a sequence of at least 3 randomized nucleotides,
c) a core sequence of at least 8 core nucleotides which sequence contains at least two mismatches compared to a target sequence,
d) a sequence of at least 3 randomized nucleotides, and
e) a 3'modification,
wherein each subset comprises a plurality of nucleic acid molecules having an identical core nucleotide sequence and different randomized nucleotides, and
wherein the nucleic acid molecules of each subset differ in at least one nucleotide of the core nucleotide sequence.

In a preferred embodiment, the core sequence is not complementary to the target sequence. Specifically, the core sequence does not hybridize with or bind to the target sequence.

The 5'phosphate can be a monophosphate, diphosphate or triphosphate.

The 3'modification can be 2'-O-methylation, i.e. a 2'-O-methyl group is coupled to the 3'end or a hydroxylation, i.e. a hydroxyl group is coupled to the 3'end of the oligonucleotide.

Although the core nucleotide sequence can be of any length appropriate to be used as spike-in for sequencing, it is preferred that the length is 8 to 25 nucleotides, preferably 10 to 20 nucleotides, preferably from 12 to 18 nucleotides, preferably 12, 13, 14, 15, 16, 17, or 18 nucleotides.

In a specific embodiment, a subset according to the invention specifically comprises nucleic acid molecules of following general formula from 5'to 3'direction:

p-(N)ₘ(x)ₙ(N)ₘ-2'-O-methyl,

wherein
p is monophosphate,
N is a random nucleotide of any of A, U, G, C;
X is any of A, U, G, C;
m is 3, 4 or 5,
n is 8, 9, 10, 11, 12, 13, 14, 15, 16, 17, 18, 19, or 20.

In a specific embodiment, a subset according to the invention specifically comprises nucleic acid molecules of following general formula from 5'to 3'direction:

p-(N)ₘ(x)ₙ(N)ₘ-2'-O-methyl,

wherein
p is monophosphate,
N is a random nucleotide of any of A, T, G, C;
X is any of A, T, G, C;
m is 3, 4 or 5,
n is 8, 9, 10, 11, 12, 13, 14, 15, 16, 17, 18, 19, or 20.

In a further specific embodiment, a subset according to the invention specifically comprises nucleic acid molecules of following general formula from 5'to 3'direction:

p-(N)ₘ(x)ₙ(N)ₘ-2'-O-methyl,

wherein
p is monophosphate,
N is a random nucleotide of any of A, U, G, C;
X is any of A, U, G, C;
m is 4
n is 8, 9, 10, 11, 12, 13, 14, 15, 16, 17, 18, 19, or 20.

In a further specific embodiment, a subset according to the invention specifically comprises nucleic acid molecules of following general formula from 5'to 3'direction:

p-(N)ₘ(x)ₙ(N)ₘ-2'-O-methyl,

wherein
p is monophosphate,
N is a random nucleotide of any of A, T, G, C;
X is any of A, T, G, C;
m is 4
n is 8, 9, 10, 11, 12, 13, 14, 15, 16, 17, 18, 19, or 20.

In a further specific embodiment, a subset according to the invention specifically comprises nucleic acid molecules of following general formula from 5'to 3'direction:

p-(N)ₘXXXXXXXXXXXXX(N)ₘ-2' -O-methyl,

wherein
p is monophosphate,
N is a random nucleotide of any of A, U, G, C;
X is independently from each other any of A, U, G, C;
m is 4.

In a further specific embodiment, a subset according to the invention specifically comprises nucleic acid molecules of following general formula from 5'to 3'direction:

p-(N)ₘXXXXXXXXXXXXX(N)ₘ-2' -O-methyl,

wherein
p is monophosphate,
N is a random nucleotide of any of A, T, G, C;
X is independently from each other any of A, T, G, C;
m is 4.

According to an embodiment of the invention, (X) or (X)₁₃ is a core sequence which sequence contains at least two mismatches compared to a target sequence, e.g. is not complementary to, or not identical, with a target sequence, specifically it is a semi random sequence.

A set comprising one or more of above listed subsets may be useful as spike-in mimicking endogenous plant small RNAs or DNAs.

Specifically, each set contains one of SEQ IDs. NO: 1 to 8.

In yet an alternative embodiment, a subset according to the invention specifically comprises nucleic acid molecules of following general formula from 5'to 3'direction:

p-(N)ₘ(x)ₙ(N)ₘ-OH

wherein
p is di- or triphosphate
N is a random nucleotide of any of A, U, G, C;
X is independently from each other any of A, U, G, C;
m is 3, 4 or 5,
n is 8, 9, 10, 11, 12, 13, 14, 15, 16, 17, 18, 19, or 20.

In a specific embodiment, a subset according to the invention specifically comprises nucleic acid molecules of following general formula from 5'to 3'direction:

p-(N)ₘ(x)ₙ(N)ₘ-OH

wherein
p is di- or triphosphate
N is a random nucleotide of any of A, T, G, C;
X is independently from each other any of A, T, G, C;
m is 3, 4 or 5,
n is 8, 9, 10, 11, 12, 13, 14, 15, 16, 17, 18, 19, or 20.

In a further specific embodiment, a subset according to the invention specifically comprises nucleic acid molecules of following general formula from 5'to 3'direction:

p-(N)ₘ(x)ₙ(N)ₘ-OH

wherein
p is di- or triphosphate
N is a random nucleotide of any of A, U, G, C;
X is independently from each other any of A, U, G, C;
m is 4
n is 8, 9, 10, 11, 12, 13, 14, 15, 16, 17, 18, 19, or 20

In a further specific embodiment, a subset according to the invention specifically comprises nucleic acid molecules of following general formula from 5'to 3'direction:

p-(N)ₘ(x)ₙ(N)ₘ-OH

wherein
p is di- or triphosphate
N is a random nucleotide of any of A, T, G, C;
X is independently from each other any of A, T, G, C;
m is 4
n is 8, 9, 10, 11, 12, 13, 14, 15, 16, 17, 18, 19, or 20.

In a further specific embodiment, a subset according to the invention specifically comprises nucleic acid molecules of following general formula from 5'to 3'direction:

p-(N)ₘXXXXXXXXXXXXX(N)ₘ-OH

wherein
p is di- or triphosphate
N is a random nucleotide of any of A, U, G, C;
X is independently from each other any of A, U, G, C;
m is 4.

In a further specific embodiment, a subset according to the invention specifically comprises nucleic acid molecules of following general formula from 5'to 3'direction:

p-(N)ₘXXXXXXXXXXXXX(N)ₘ-2' -O-methyl,

wherein
p is di- or triphosphate
N is a random nucleotide of any of A, T, G, C;
X is independently from each other any of A, T, G, C;
m is 4.

According to an embodiment of the invention, (X)ₙ or (X)₁₃ is a core sequence containing at least two mismatches compared to the target sequence, e.g. not complementary to or not identical with a target sequence, specifically it is a semi random sequence.

A set comprising one or more of above listed subsets may be useful as spike-in mimicking endogenous animal or plant small RNAs or DNAs.

Libraries of nucleic acids for use in sequencing methods can be made by mixing one or more sets containing the oligonucleotides with fragments of target nucleic acid molecules or target sequences of a desired length, converting the nucleic acid strands into double stranded DNA, attaching adapters to the ends of the fragments/oligonucleotides and quantitating the final library product for sequencing.

Reference values in nucleotide sequencing can be determined by the steps of adding the inventive set containing subsets in different amounts to a mixture of target sequences of interest, thereby generating a library of nucleic acid molecules, ligating adaptors to the nucleic acid molecules, amplifying the nucleic acid molecules of the library, sequencing the nucleic acid molecules and determining the absolute amount of the nucleic acid molecules of each subset as reference value, optionally followed by comparing the amount of target nucleic acid molecules with the reference values.

Advantageously, the libraries capture the entire transcriptome, including coding, non-coding, anti-sense and intergenic RNAs with high integrity. Alternatively the library encompasses only coding mRNA transcripts or, as a further alternative, small RNAs are profiled, e.g. miRNA, snoRNA, piRNA, snRNA and tRNA.

Small RNA are <500 nt (nucleotide) in length, and are usually non-coding RNA molecules. RNA silencing is often a function of these molecules, with the most common and well-studied example being RNA interference (RNAi), in which endogenously expressed microRNA (miRNA) or exogenously derived small interfering RNA (siRNA) induces the degradation of complementary messenger RNA. Other classes of small RNA have been identified, including piwi-interacting RNA (piRNA), small nucleolar RNA (snoRNA), tRNA-derived small RNA (tsRNA), small rDNA-derived RNA (srRNA), small nuclear RNA and its subspecies repeat associated small interfering RNA (rasiRNA), Signal recognition particle RNA (SRP RNA), 7SK small nuclear RNA, RNase and MRP RNA.

When preparing a sequencing library, it is a main objective to reduce bias.

Bias can be defined as the systematic distortion of data due to the experimental design. The present set and its use advantageously highly minimizes or eliminates bias. By providing a method wherein absolute numbers, e.g. numbers of molecules, can be determined, it is possible to compare small RNA sequence data from various tissues with dynamic small RNA populations or mutations that alter these populations.

The inventive spike-ins and method is thus specifically advantageous for comparing the copy number of small RNA molecules from different cell types. Thereby, for example, sRNA sub-populations from different organs or tissues or compartments can be compared and said method can be used for data normalization. Specifically, absolute normalization of sRNA-sequence data from tissues, organs or compartments with different smallRNA populations can be determined, thus enabling accurate comparisons of total and individual sRNA levels.

The invention provides for a new method for determining the number of nucleic acid molecules in a sample, wherein the inventive oligonucleotide set is added to the sample to get a mixture of nucleic acid molecules, thereby generating a library of nucleic acid molecules, adaptors are ligated to the nucleic acid molecules of the library, the library is optionally amplified and a Next Generation Sequencing of said amplified library is performed thereby resulting in RNA sequence reads from said nucleic acid molecules, and the number of reads from the set and from the sample are determined. The number of reads of the set may then be aligned to the target sequence from a genome of interest and reads containing identical core sequences are optionally grouped together for further analysis.

The amount of one or more target sequences in the sample can be determined and optionally relative quantification of the nucleic acid molecules of a sample (e.g. reads per million) or absolute quantification of the nucleic acid molecules in a sample (molecules per microgram) can be determined.

The method for determining reference values of the invention can further be used for normalizing sRNA sequence data from different source and for assessing cloning biases occurring during sRNA library preparation.

The present invention further encompasses following items:
1. A set comprising at least two subsets of single stranded nucleic acid molecules, each nucleic acid molecule comprising from the 5'to 3'direction:
   a) a 5'phosphate,
   b) a sequence of at least 3 randomized nucleotides,
   c) a core sequence of at least 8 nucleotides containing two or more mismatches compared to a target sequence,
   d) a sequence of at least 3 randomized nucleotides, and
   e) a 3'modification,
   wherein each subset comprises a plurality of nucleic acid molecules having an identical core nucleotide sequence and different randomized nucleotides, and wherein the nucleic acid molecules of each subset differ in at least one nucleotide of the core nucleotide sequence.
2. A set comprising at least two subsets of single stranded nucleic acid molecules, each nucleic acid molecule comprising from the 5'to 3'direction:
   a) a 5'phosphate,
   b) a sequence of at least 3 randomized nucleotides,
   c) a core sequence of at least 8 nucleotides containing two or more mismatches compared to a target sequence,
   d) a sequence of at least 3 randomized nucleotides, and
   e) a 3'modification,
   wherein each subset comprises a plurality of nucleic acid molecules having an identical core nucleotide sequence and different randomized nucleotides, and wherein the nucleic acid molecules of each subset differ in at least one nucleotide of the core nucleotide sequence.
3. A set comprising at least two subsets of single stranded nucleic acid molecules, each nucleic acid molecule consisting of:
   a) a 5'phosphate,
   b) a sequence of at least 3 randomized nucleotides,
   c) a core sequence of at least 8 nucleotides containing two or more mismatch compared to a target sequence,
   d) a sequence of at least 3 randomized nucleotides, and
   e) a 3'modification,
   wherein each subset comprises a plurality of nucleic acid molecules having an identical core nucleotide sequence and different randomized nucleotide sequences, and wherein the nucleic acid molecules of each subset differ in at least one nucleotide of the core nucleotide sequence.
4. The set according to any of items 1 to 3, wherein the randomized nucleotide sequences are any one of A, C, G, U or A, C, G, T.
5. The set according to any one of items 1 to 4, wherein the plurality of nucleic acid molecules comprises randomized nucleotide sequences containing all four nucleotide combinations of A, C, G, U or A, C, G, T.
6. The set according to any one of items 1 to 5, wherein the nucleic acid molecule is an RNA molecule, specifically mimicking a small RNA.
7. The set according to any one of items 1 to 6, wherein the small RNA is selected from the group consisting of siRNA, tasiRNA, snRNA, miRNA, snoRNA, piRNA and tRNA or any precursors thereof.
8. The set according any one of items 1 to 7, wherein the core nucleotide sequence comprises from 8 to 25 nucleotides, preferably from 10 to 20 nucleotides, preferably from 12 to 18 nucleotides, preferably 13 nucleotides.
9. The set according to any one of items 1 to 8, wherein the core nucleotide sequences of each subset differ in 1, 2, 3, 4, 5, 6, 7, 8, 9, 10 or more nucleotides.
10. The set according to any one of items 1 to 9, wherein the core nucleotide sequences are semi random sequences.
11. The set according to any one of items 1 to 10, wherein the core nucleotide sequence is different from the genome or transcriptome of an organism of interest.
12. The set according to any one of items 1 to 11, wherein the sequence of randomized nucleotides comprises from 3 to 7 nucleotides, preferably from 3 to 5 nucleotides, preferably 4 nucleotides.
13. The set according to any one of items 1 to 12, wherein the 5'phosphate is selected from a group of monophosphate, diphosphate and triphosphate.
14. The set according to any one of items 1 to 13, wherein the 3'modification is selected from a group consisting of 2'-O-methylation and hydroxylation.
15. The set according to any one of items 1 to 14, comprising 3, 4, 5, 6, 7, 8, 9, 10, 11, 12, 13, 14, 15, 16, 17, 18, 19, 20, 21, 22, 23, 24 or more subsets.
16. The set according to any one of items 1 to 15, wherein the subsets are present in an amount from 1 to 10000 amol, preferably from 10 to 5000 amol.
17. The set according to any one of items 1 to 16, comprising different amounts of each subset.
18. The set according to any one of items 1 to 17, wherein the target sequence can be any sequence of interest, specifically a genome or transcriptome of an organism, a sequence originating from virus, bacteria, animals, plants, specifically an RNA, specifically a small RNA, specifically a dynamic small RNA.
19. Use of a set according to any one of items 1 to 18 as spike-in probes for normalizing sequencing data.
20. A method for determining the absolute amount of one or more target sequences in a sample, specifically in a cell, tissue or organ sample using a set according to any one of items 1 to 18.
21. A method for generating a library of nucleic acids for use in sequencing methods, wherein one or more sets according to any one of items 1 to 18 are mixed with target nucleic acid molecules.
22. The method of item 21, wherein the nucleic acid library comprises small RNAs.
23. The method of item 21 or 22, further amplifying the library.
24. A method for determining reference values in nucleotide sequencing, comprising the steps
   - adding a set according to any one of items 1 to 18 to a mixture of target sequences, thereby generating a library of nucleic acid molecules,
   - ligating adaptors to the library
   - optionally amplifying said library,
   - performing a nucleotide sequencing method,
   - determining the amount of nucleic acid molecules of each subset as reference value.
25. A method for determining the absolute amount of a target sequence, wherein the amount of the target sequences is compared with the reference values obtained by the method of item 24.
26. The method according to item 25, wherein the copy number of small RNA molecules from different cell types are compared.
27. A method for determining the number of nucleic acid molecules in a sample, comprising the steps of
   a) adding a set according to any one of items 1 to 18 to the sample to get a mixture of nucleic acid molecules, thereby generating a library of nucleic acid molecules,
   b) amplifying said library,
   c) performing Next Generation Sequencing of said library resulting in RNA sequence reads from said nucleic acid molecules,
   d) determining the number of reads from the set and from the sample.
28. The method of item 26, wherein the number of reads of the set is aligned to the target sequence, e.g. from a genome of interest, and reads containing identical core sequences are optionally grouped together for further analysis
29. The method according to any one of items 24 to 28 for determining the amount of one or more target sequences in the sample.
30. The method according to any one of items 24 to 28 for absolute quantification of the small RNA molecules in a sample.
31. The method according to any one of items 24 to 28 for normalizing sRNA sequence data from different sources.
32. Use of the method according to any one of items 24 to 28 for assessing cloning biases occurring during sRNA library preparation.
33. An oligonucleotide described by the general formula:

   p-(N)ₘ(x)(N)ₘ-2'-O-methyl

   , wherein
   N is a random nucleotide of any of A, U, G, C;
   X is a core sequence containing one or more mismatches compared to a target sequence and having a length of 8, 9, 10, 11, 12, 13, 14, 15, 16, 17, or 18 nucleotides comprising any of A, U, G, C;
   m is 3, 4 or 5.
34. A method for preparing spike-ins for sequencing, wherein at least two different oligonucleotides according to item 33 are used.
35. The method of item 34, wherein the spike-ins comprise a plurality of oligonucleotides of item 32 containing all possible nucleotide combinations of A, U, C, G.

The present invention is further illustrated by the following examples without being limited thereto.

### Examples

### Methods

### Plant material

The *dcl234* mutants were composed of *dcl2-1, dcl3-1* and *dcl4-2t* alleles as previously described¹⁷. Plants were grown in a controlled growth chamber at 20ºC-22ºC with a 16-h light/8-h dark cycle. Col-0 leaf samples were from rosette and cauline leaves isolated from 4-6 week old plants. Unopened floral buds were either collected from the same plants as the leaf samples (Col-0 flower) or from *dcl234* plants (*dcl234* flowers) grown under identical conditions.

### sRNA spike-in design

A matrix consisting of the proportions of base identities for miRNA positions 5-17 (counting from the 5' end of the miRNA) of the top 50 most highly abundant miRNAs was generated, and used to semi-randomly select 1000 13 nt sequences. Two hundred and fifty-two sequences that did not perfectly align to the *Arabidopsis thaliana* Col-0 genome were considered further and all 256 possible 4-base combinations were added to both the 5' and 3' ends making 65,536 total sequences per set. The minimum free energies of all 21 nt RNAs within the 252 sets of 65,536 sequences were determined using RNAfold²⁵. Minimum free energy distributions were then examined and eight sets of 21 nt RNA sequences with distributions similar to annotated miRNAs were selected for synthesis and ordered from Integrated DNA Technologies (IDT). The mix ratios were formulated to span the dynamic range of annotated *Arabidopsis* miRNAs.

Design of small RNA spike-in oligonucleotides. Key features of small RNA spike-ins are shown in in bold or italic corresponding to the key. Molar amounts of oligonucleotides added per µg of total RNA are indicated in parentheses.

| | | |
|---|---|---|
| **p-***NNNN*GAGUCAUGCAUUA*NNNN***-**2'**-O-methyl** | SEQ ID NO: 1 | (10 amol) |
| **p-***NNNN*UAUGCCAGAAGUC*NNNN*-**2'-O-methyl** | SEQ ID NO: 2 | (50 amol) |
| **p-***NNNN*UCUAACGUGCCUA*NNNN*-**2'-O-methyl** | SEQ ID NO: 3 | (100 amol) |
| **p-***NNNN*UGCGCAGACAUAA*NNNN***-2'-O-methyl** | SEQ ID NO: 4 | (300 amol) |
| **p-***NNNN*AAGCGAUUACGAC*NNNN*-**2'-O-methyl** | SEQ ID NO: 5 | (500 amol) |
| **p-***NNNN*AUGUGAAUGACCG*NNNN***-2'-O-methyl** | SEQ ID NO: 6 | (700 amol) |
| **p-***NNNN*GUACCCAUGUGAA*NNNN***-2'-O-methyl** | SEQ ID NO: 7 | (1000 amol) |
| **p-***NNNN*UAUUGAUCGCGCU*NNNN***-2'-O-methyl** | SEQ ID NO: 8 | (5000 amol) |

RNA oligos with 5' phosphate and 2'-O-methyl groups mimic endogenous small RNAs; this example would be for typical plant small RNAs and animal siRNAs, but the 2'-O-me group could be replaced with hydroxyl groups to mimic canonical animal microRNAs

Four randomized nucleotides at 5' and 3' terminals, -each oligo has 4⁸ (65,536) possible combinations, which allows the generation of accurate standard curves (for absolute molecule quantification) and detection of cloning biases

Semi-random non-genome-matching core 13mer. The above design was for Arabidopsis, and likely suitable for many other plants, but this could be adapted for any organism

### RNA sequencing

The sRNA spike-in mix shown in Fig. 1a was diluted two-fold and added to 500 ng of total RNA prior to polyacrylamide gel size-selection followed by sRNA cloning using the NEBnext small RNA library prep kit for Illumina (NEB). ERCC spike-in mixes (LifeTech) were diluted 200-fold and 1 µl was added to 500 ng of total RNA. Ten ng of total RNA was used to generate mRNA-Seq libraries as described by Picelli et al.²⁶. Samples were sequenced on an Illumina Hi-Seq 2500 sequencing machine in either single read 50 bp (sRNA-Seq) or paired-end read 50 bp (mRNA-Seq) modes.

### Data analysis

After removing adaptor sequences, sRNA-Seq reads were aligned to the *Arabidopsis thaliana* Col-0 genome (TAIR10) with sRNA spike-ins using the Bowtie short-read aligner²⁷ requiring perfect matches and allowing up to 100 alignments per read (**Tables 1** and **2**). Reads containing common 13 nt sequences of the sRNA spike-ins were then grouped together for further analysis.

**Table 1: Statistics for sRNA-Seq libraries**

| **Library^{a}** | **Col-0 leaf #1** | **Col-0 leaf #2** | **Col-0 flowers #1** | **Col-0 flowers #2** | ***dcl234* flowers #1** | ***dcl234* flowers #2** |
|---|---|---|---|---|---|---|
| Genome-matching reads^{b} | 8,786,408 | 4,062,090 | 13,243,396 | 13,755,078 | 10,164,548 | 8,590,559 |
| sRNA spike-in matching reads | 451,446 | 303,113 | 261,989 | 274,476 | 150,279 | 147,114 |
| % sRNA spike-in reads | 5.14% | 7.46% | 1.98% | 1.99% | 1.48% | 1.71% |

| | | | | | | |
|---|---|---|---|---|---|---|
| ^{a} #1 and #2 indicate biological replicates 1 and 2, respectively ^{b} genome-matching reads also include the number of reads that map to either ERCC or sRNA spike-ins | | | | | | |

**Table 2: Statistics for mRNA-Seq libraries**

| **Library^{a}** | **Col-0 leaf #1** | **Col-0 leaf #2** | **Col-0 flowers #1** | **Col-0 flowers #2** | ***dcl234* flowers #1** | ***dcl234* flowers #2** |
|---|---|---|---|---|---|---|
| Genome-matching reads^{b} | 9204152 | 9156745 | 14357321 | 11836228 | 12193753 | 14294965 |
| mRNA spike-in matching reads | 19072 | 8199 | 29000 | 21057 | 10354 | 5391 |
| % sRNA spike-in reads | 0.21% | 0.09% | 0.20% | 0.18% | 0.08% | 0.04% |

| | | | | | | |
|---|---|---|---|---|---|---|
| ^{a} #1 and #2 indicate biological replicates 1 and 2, respectively ^{b} genome-matching reads also include the number of reads that map to ERCC spike-ins | | | | | | |

Small RNA-Seq reads were then assigned to mature miRNAs or tasiRNAs if they were 20-22 nt long and contained within ± 2 nt of the sense strand of the miRNA and tasiRNA according to annotations in miRBase21²⁸ and Allen *et al*.²⁹, respectively. Values for individual miRNAs or tasiRNAs belonging to common families were then added together to obtain the total amount of reads for individual families. Small RNA reads which were either 20-22 nt or 23-24 nt long and overlapped either strand of TAIR10 annotated transposons (i.e. transposable elements and transposable element genes) were grouped according to the transposon that they mapped to. Paired-end mRNA-Seq reads were aligned to the *Arabidopsis thaliana* Col-0 genome (TAIR10) and ERCC spike-ins using RSEM³⁰. Reads with up to 20 alignments were retained and assigned to ERCC spike-ins or transcript models based on Araport11 annotations³¹. Statistical analyses and graphics were performed with R³².

To generate a set of exogenous sRNA spike-ins for absolute normalization of sRNA-Seq data, we designed 21 nucleotide (nt) RNA oligonucleotides with three main features (Fig. 1a). First, the sRNA spike-ins contained a 5' monophosphate and 2'-O-methyl group in order to mimic endogenous plant small RNAs. The 2'-O-methyl group is common to plant small RNAs¹⁴, but this modification could be omitted if investigating animal miRNAs for instance. Second, the sRNA spike-ins contained a semi-random 13 nt core sequence that does not match the genome-of-interest (e.g. in this study *Arabidopsis thaliana*). Third, these semi-random 13 nt core sequences are flanked by a set of four randomized nucleotides on both the 5' and 3' ends. Eight sRNA spike-ins with different 13 nt core sequences were designed and mixed in specific molar ratios as shown in Fig. 1a. These were added to total RNA prior to sRNA-Seq library preparation. After sequencing, the non-genome matching 13 nt unique tags were used to quantify reads derived specifically from each sRNA spike-in. Each of these 13 nt core sequences can be represented by up to 65,536 possible 21 nt sequences. Because individual sRNA sequences have variable properties, such as secondary structure, that bias their representation in the final sRNA-Seq libraries¹¹⁻¹³, the large number of diverse sequences that can be assigned to each 13 nt core sequence of the sRNA spike-in is expected to minimize cloning biases of each spike-in set as a whole. Therefore, the sRNA spike-ins enabled the robust generation of standard curves for absolute data normalization in terms of molecules per µg total RNA (MPU) (Fig. 1b). Nearly perfect positive correlations (all had Pearson's r values ≥ 0.99 and *P* < 7.42 x 10⁻⁶) were observed when plotting relative RPM levels reported by sRNA-Seq and the known absolute MPU amounts of sRNA spike-ins added to each sample (Fig. 1b). As a proof of concept, the highly linear relationships between relative and absolute values were used to generate linear models to predict the number of miRNA molecules per µg of total RNA isolated from wild-type (Col-0) flowers (Fig. 1c).

Because the sRNA spike-ins enabled accurate data normalization (Fig. 1), we compared sRNA sub-populations in relative RPM and absolute MPU terms to determine if the two normalization procedures yield different results. Although similar analyses could be performed on any species, we used *Arabidopsis thaliana* to test the utility of the sRNA spike-ins because of its well-annotated sRNAs and transcripts, readily available different tissue types and viable mutants deficient in major sRNA sub-populations. Plant sRNA populations consist of four main classes: 20-22 nt microRNAs (miRNAs) and trans-acting siRNAs (tasiRNAs) that tend to post-transcriptionally regulate protein-coding genes, and 20-22 nt and 23-24 nt small interfering RNAs (siRNAs) that typically arise from and silence transposons¹⁵. The proportions of sRNA sub-populations vary between flowers and leaves (Fig. 4), which makes comparisons of relatively normalized leaf and flower sRNA levels prone to errors. For example, 30% and 53% of the respective Col-0 flower and Col-0 leaf sRNA populations were composed of 20-22 nt sequences (Fig. 4a-c), but it cannot be determined whether there are absolutely more 20-22 nt sRNAs in leaves or whether this relative increase is merely due to reduced 23-24 nt siRNA levels in leaves compared to flower tissues. Using sRNA spike-ins, we compared relative and absolute normalization methods on sRNA sub-populations in Col-0 flowers and leaves. The two normalization methods produced different results. For example, whereas total miRNA levels have a significantly higher RPM in Col-0 leaves compared to Col-0 flowers (1.7-fold; *P* = 3.2 x 10⁻³; two-sample Student's t-test), the absolute number of miRNAs are non-significantly reduced 1.5-fold in Col-0 leaves compared to Col-0 flowers (*P* = 0.13; two-sample Student's t-test) (Fig. 4e, f). Moreover, absolute, but not relative, normalization shows that miRNA families have significantly increased levels in Col-0 flowers compared to Col-0 leaves (Fig. 2a, b) (*P* = 4.1 x 10⁻³; two-sample Kolmogorov-Smirnov test). Therefore, relative normalization of sRNA-Seq data followed by comparisons between tissue types can lead to misleading results, which are mitigated through the use of sRNA spike-ins.

To further test how changes in sRNA populations can affect relative normalization, we generated sRNA-Seq libraries from siRNA-deficient flowers and compared these to the Col-0 flower datasets. More specifically, we generated sRNA-Seq libraries from flowers with null mutations in three genes encoding the DICER-LIKE2 (DCL2), DCL3 and DCL4 ribonucleases (i.e *dcl234* flowers). *DCL3* and *DCL4* are required for 23-24 nt siRNA and tasiRNA biogenesis, respectively¹⁶⁻²⁰ (Fig. 4c). Therefore, *dcl234* flowers are expected to have reduced tasiRNA and 23-24 nt siRNA levels. Comparisons of relative and absolute normalization methods on 20-24 nt siRNA levels produced similar results (Fig. 4d, e and Fig. 2a, b). However, relative RPM levels for total tasiRNAs were not significantly reduced in *dcl234* flowers compared to Col-0 flowers; whereas, absolute MPUs for total tasiRNAs were significantly reduced (*P*_{RPM} = 0.08, *P*_{MPU} = 0.04; two-sample Student's t-test) (Fig. 4d, e). Both relatively and absolutely normalized tasiRNA family levels are significantly reduced in *dcl234* flowers but the decrease is more pronounced when using absolute terms (*P*_{RPM} = 1.0 x 10⁻³, *P*_{MPU} = 1.6 x 10⁻⁴; two-sample Kolmogorov-Smirnov test) (Fig. 2a, b). We also found that the total amount of miRNAs had both significantly higher RPMs and MPUs in *dcl234* flowers compared to Col-0 flowers (*P*_{RPM} = 0.04, *P*_{MPU} = 0.02; two-sample Student's t-tests) (Fig. 4a, b). Therefore, in the absence of siRNAs, miRNAs are increased as has been previously proposed²¹.

The levels of individual sRNAs are often compared across sRNA-Seq datasets from various tissue types in order to determine when and where a particular sRNA is most abundant. We compared relatively and absolutely normalized values for 93 miRNA families in Col-0 flowers versus Col-0 leaves or *dcl234* flowers to determine if the two normalization methods yield different results. Indeed, comparisons of RPM-based values suggest that 13 and 6 miRNA families have respectively increased and decreased levels in Col-0 leaves compared to Col-0 flowers (Fig. 2c). In contrast, due to the higher absolute miRNA levels in flowers (Fig. 4e and Fig. 2b), comparisons of MPU-based values indicate that no miRNA families are increased in Col-0 leaves compared to flowers, and 19 miRNA families are decreased in Col-0 leaves (Fig. 2d). Moreover, 35 miRNA families have increased levels in *dcl234* versus Col-0 flowers based on RPM values; whereas, only 16 miRNA families are increased in *dcl234* flowers compared to Col-0 flowers when using MPU values for the comparisons (Fig. 2c, d). Based on our results, absolute normalization of sRNA-Seq data from tissues with different underlying small RNA populations enables more accurate comparisons of both total and individual sRNA levels.

The relationships between sRNA levels and the abundance of either their precursors or targets are important to understand miRNA biogenesis and function. Because small RNAs and their longer RNA precursors/targets require different procedures for selection and cloning into RNA-Seq libraries, it is impossible to use relative normalization approaches to estimate the stoichiometries between sRNAs and their precursors or targets. We tested whether sRNA spike-ins together with commercially available mRNA spike-ins (ERCC spike-in mixes; LifeTech) enable cross-comparisons between sRNA-Seq and mRNA-Seq datasets. More specifically, we generated mRNA-Seq datasets from the same total RNA samples used to generate the sRNA-Seq data described above, and added ERCC spike-in mixes to the total RNA prior to mRNA-Seq library construction. Strong positive correlations between relative numbers of ERCC transcripts (transcripts per kilobase million; TPM) and the known number of molecules added per µg of total RNA were observed for all six datasets having Pearson's *r* values of at least 0.96 and *P* < 1.44 x 10⁻¹² (Figs 3a and Fig. 5a-e). We then used these relationships to generate linear models and applied these to mRNAs with TPMs ≥ 1.0 in order to estimate mRNA MPUs genome-wide in all six datasets. This allowed us to then compare mature sRNA MPUs with those from either their precursors or targets. We found that miRNA/precursor ratios were similar in Col-0 leaves, Col-0 flowers and *dcl234* flowers with respective median miRNA/precursor levels of 2.1, 1.6 and 1.5 (Fig. 3b). The ratios between tasiRNAs and their precursors were higher, but not significantly different, than miRNA/precursor ratios in Col-0 leaves and Col-0 flowers with median tasiRNA/precursor ratios of 3.7 and 4.4, respectively (Fig. 3b). In contrast, tasiRNA precursors were more abundant than mature tasiRNA levels in *dcl234* flowers with a median of 42.4-fold more precursors than tasiRNAs (Fig. 3b). This is consistent with the known requirement of *DCL4* for tasiRNA biogenesis and the corresponding increase in tasiRNA precursor levels in *dcl234* tissues^{16-18,20,22}.

We then investigated the stoichiometries between miRNAs and their targets genome-wide. We used publically available datasets of sRNA cleavage products (i.e. degradome datasets) from Col-0 flowers to select miRNA and tasiRNA targets^{23,24}. Ratios between miRNAs and their targets were not significantly different in Col-0 and *dcl234* flowers and had median values of 3.9 and 0.63, respectively. In contrast, tasiRNA/target ratios were significantly different in Col-0 and *dcl234* flowers as expected (*P* = 5.0e⁻¹⁶; two-sample Kolmogorov-Smirnov test) with respective 1.5-fold and 210.8-fold more target MPUs compared to tasiRNA MPUs (Fig. 3c). Therefore, in combination with mRNA spike-ins, sRNA spike-ins allow genome-wide estimations of precursor:sRNA and sRNA:target stoichiometries.

Small RNA spike-ins not only serve as useful internal controls for sRNA-Seq experiments requiring only 1-2% of the alignable reads for subsequent analyses, but can also be used to normalize sRNA-Seq data from different treatments, tissue types or research groups. Moreover, they enable the absolute quantification of sRNA molecules, which can be essential for accurate comparisons, as well as genome-wide estimations of precursor:sRNA and sRNA:target stoichiometries, which are important for understanding these relationships on a molecular scale. Lastly, sRNA spike-ins could be used to assess and improve upon cloning biases that exist in various sRNA-Seq library generation protocols.

### References

1. Meyer, S. U., Pfaffl, M. W. & Ulbrich, S. E. Biotechnol Lett 32, 1777-1788 (2010).
2. Malone, C. D. etal. Cell 137, 522-535 (2009).
3. Farh, K. K. et al. Science 310, 1817-1821 (2005).
4. Stark, A., Brennecke, J., Bushati, N., Russell, R. B. & Cohen, S. M. Cell 123, 1133-1146 (2005).
5. Breakfield, N. W. et al. Genome Research 22, 163-176 (2012).
6. Rajagopalan, R., Vaucheret, H., Trejo, J. & Bartel, D. P. Genes & Development 3407-3425 (2006).
7. Martinez, G., Panda, K., Kohler, C. & Slotkin, R. K. Nature Plants 1-8 (2016).
8. Fahlgren, N. et al. RNA 15, 992-1002 (2009).
9. Powers, J. T. et al. Nature 535, 246-251 (2016).
10. Locati, M. D. etal. Nucleic Acids Research 43, e89-e89 (2015).
11. Hafner, M. etal. RNA 17, 1697-1712 (2011).
12. Jayaprakash, A. D., Jabado, O., Brown, B. D. & Sachidanandam, R. Nucleic Acids Research 39, e141 -e141 (2011).
13. Sorefan, K. et al. Silence 3, 1-1 (2012).
14. Yu, B. et al. Science 307, 932-935 (2005).
15. Matzke, M. A. & Mosher, R. A. Nature Reviews Genetics 15, 394-408 (2014).
16. Gasciolli, V., Mallory, A. C., Bartel, D. P. & Vaucheret, H. Current Biology 15, 1494-1500 (2005).
17. Henderson, I. R. et al. Nat Genet 38, 721-725 (2006).
18. Howell, M. D. et al. The Plant Cell 19, 926-942 (2007).
19. Yoshikawa, M. Genes & Development 19, 2164-2175 (2005).
20. Xie, Z. et al. PLoS Biology 2, 0642-0652 (2004).
21. Yu, B. et al. Nucleic Acids Research 38, 5844-5850 (2010).
22. Yoshikawa, M., Peragine, A., Park, M. Y. & Poethig, R. S. Genes & Development 19, 2164-2175 (2005).
23. Addo-Quaye, C., Eshoo, T. W., Bartel, D. P. & Axtell, M. J. Current Biology 18, 758-762 (2008).
24. Addo-Quaye, C., Miller, W. & Axtell, M. J. Bioinformatics 25, 130-131 (2008).
25. Lorenz, R. et al. Algorithms for Molecular Biology 6, 1-14 (2011).
26. Picelli, S. et al. Nat Meth 10, 1096-1098 (2013).
27. Langmead, B., Trapnell, C., Pop, M. & Salzberg, S. L. Genome Biol 10, R25 (2009).
28. Kozomara, A. & Griffiths-Jones, S. Nucleic Acids Research 42, D68-D73 (2013).
29. Allen, E., Xie, Z., Gustafson, A. M. & Carrington, J. C. Cell 121, 207-221 (2005).
30. Li, B. & Dewey, C. N. BMC Bioinformatics 12, 323 (2011).
31. Cheng, C.-Y., Krishnakumar, V., Chan, A., Schobel, S. & Town, C. D. (2016). doi:10.1101/047308
32. R Core Team. www.R-project.org (2016). at <http://www.R-project.org/>
33. Raabe, C., Tang, T-H., Brosius J., Rozhdestvensky T.S., Nucleic Acid Research, 42, 3, 1414-1426 (2014)

## Claims

1. A set comprising at least two subsets of single stranded nucleic acid molecules, each nucleic acid molecule comprising from the 5'to 3'direction:
a) a 5'phosphate,
b) a sequence of at least 3 randomized nucleotides,
c) a core sequence of at least 8 nucleotides which sequence contains two or more mismatches compared to a target sequence,
d) a sequence of at least 3 randomized nucleotides, and
e) a 3'modification,
wherein each subset comprises a plurality of nucleic acid molecules having an identical core nucleotide sequence and different randomized nucleotides, and
wherein the nucleic acid molecules of each subset differ in at least one nucleotide of the core nucleotide sequence.

2. The set according to claim 1, wherein the plurality of nucleic acid molecules comprises randomized nucleotide sequences containing all four nucleotide combinations of A, C, G, U or A, C, G, T.

3. The set according to any one of claims 1 to 2, wherein the nucleic acid molecule is an RNA molecule, specifically mimicking a small RNA, specifically selected from the group consisting of siRNA, tasiRNA, snRNA, miRNA, snoRNA, piRNA and tRNA and any precursors thereof.

4. The set according to any one of claims 1 to 3, wherein the core nucleotide sequence comprises from 8 to 25 nucleotides, preferably from 10 to 20 nucleotides, preferably from 12 to 18 nucleotides, preferably 13 nucleotides.

5. The set according to any one of claims 1 to 4, wherein the sequence of randomized nucleotides comprises from 3 to 7 nucleotides, preferably from 3 to 5 nucleotides, preferably 4 nucleotides.

6. The set according to any one of claims 1 to 5, wherein the 5'phosphate is selected from a group of monophosphate, diphosphate and triphosphate and wherein the 3'modification is selected from a group consisting of 2'-O-methylation [2'-O-methyl group] and hydroxylation [hydroxyl group].

7. The set according to any one of claims 1 to 6, wherein the subsets are present in an amount from 1 to 10000 amol, preferably from 10 to 5000 amol, specifically comprising different amounts of each subset.

8. The set according to any one of claims 1 to 7, wherein the target sequence can be any sequence of interest, specifically a genome or transcriptome of an organism, a sequence originating from virus, bacteria, animals, plants, specifically it is an RNA small RNA, dynamic small RNA population.

9. Use of a set according to any one of claims 1 to 8 as spike-in probes for normalizing sequencing data.

10. A method for determining the absolute amount of one or more target sequences in a sample, specifically in a cell, tissue or organ sample using a set according to any one of claims 1 to 9.

11. A method for determining reference values in nucleotide sequencing, comprising the steps
- adding a set according to any one of claims 1 to 9 to a mixture of target sequences, thereby generating a library of nucleic acid molecules,
- ligating adaptors to the library,
- optionally amplifying said library,
- performing a nucleotide sequencing method,
- determining the amount of nucleic acid molecules of each subset as reference value.

12. The method according to claim 11, wherein the copy number of small RNA molecules from different cell types are compared.

13. A method for determining the number of nucleic acid molecules in a sample, comprising the steps of
a) adding a set according to any one of claims 1 to 9 to the sample to get a mixture of nucleic acid molecules, thereby generating a library of nucleic acid molecules,
b) optionally amplifying said library,
c) performing Next Generation Sequencing of said library resulting in RNA sequence reads from said nucleic acid molecules,
d) determining the number of reads from the set and from the sample.

14. Use of the method according to any one of claims 10 to 13 for assessing cloning biases occurring during sRNA library preparation.

15. An oligonucleotide of general formula:
p-(N)ₘ(x)(N)ₘ-2'-O-methyl,
wherein
N is a random nucleotide of any of A, U, G, C;
X is a core sequence containing at least two mismatches compared to a target sequence having a length of 8, 9, 10, 11, 12, 13, 14, 15, 16, 17, or 18 nucleotides comprising any of A, U, G, C;
m is 3, 4 or 5.

## Patentansprüche

1. Set, das mindestens zwei Teilsets von einzelsträngigen Nukleinsäuremolekülen umfasst, wobei jedes Nukleinsäuremolekül in der 5'- nach 3'-Richtung Folgendes umfasst:
a) ein 5'-Phosphat,
b) eine Sequenz von mindestens 3 randomisierten Nukleotiden,
c) eine Kernsequenz von mindestens 8 Nukleotiden, wobei die Sequenz zwei oder mehr Fehlpaarungen im Vergleich zu einer Zielsequenz enthält,
d) eine Sequenz von mindestens 3 randomisierten Nukleotiden, und
e) eine 3'-Modifikation,
wobei jedes Teilset eine Vielzahl von Nukleinsäuremolekülen mit einer identischen Kernnukleotidsequenz und verschiedenen randomisierten Nukleotiden umfasst, und
wobei sich die Nukleinsäuremoleküle jedes Teilsets in mindestens einem Nukleotid der Kernnukleotidsequenz unterscheiden.

2. Set nach Anspruch 1, wobei die Vielzahl von Nukleinsäuremolekülen randomisierte Nukleotidsequenzen umfasst, die alle vier Nukleotidkombinationen von A, C, G, U oder A, C, G, T enthalten.

3. Set nach einem der Ansprüche 1 bis 2, wobei das Nukleinsäuremolekül ein RNA-Molekül ist, das insbesondere eine kleine RNA nachahmt, insbesondere ausgewählt aus der Gruppe bestehend aus siRNA, tasiRNA, snRNA, miRNA, snoRNA, piRNA und tRNA und beliebigen Vorläufern davon.

4. Set nach einem der Ansprüche 1 bis 3, wobei die Kernnukleotidsequenz 8 bis 25 Nukleotide, vorzugsweise 10 bis 20 Nukleotide, vorzugsweise 12 bis 18 Nukleotide, vorzugsweise 13 Nukleotide umfasst.

5. Set nach einem der Ansprüche 1 bis 4, wobei die Sequenz randomisierter Nukleotide 3 bis 7 Nukleotide, vorzugsweise 3 bis 5 Nukleotide, vorzugsweise 4 Nukleotide umfasst.

6. Set nach einem der Ansprüche 1 bis 5, wobei das 5'-Phosphat aus einer Gruppe von Monophosphat, Diphosphat und Triphosphat ausgewählt ist und wobei die 3'-Modifikation aus einer Gruppe bestehend aus 2'-O-Methylierung [2'-O-Methylgruppe] und Hydroxylierung [Hydroxylgruppe] ausgewählt ist.

7. Set nach einem der Ansprüche 1 bis 6, wobei die Teilsets in einer Menge von 1 bis 10.000 amol, vorzugsweise von 10 bis 5.000 amol vorhanden sind, insbesondere umfassend unterschiedliche Mengen jedes Teilsets.

8. Set nach einem der Ansprüche 1 bis 7, wobei die Zielsequenz eine beliebige Sequenz von Interesse sein kann, insbesondere ein Genom oder Transkriptom eines Organismus, eine Sequenz, die von Viren, Bakterien, Tieren, Pflanzen abstammt, wobei es insbesondere eine RNA-, kleine RNA-, dynamische kleine RNA-Population ist.

9. Verwendung eines Sets nach einem der Ansprüche 1 bis 8 als Spike-Sonden zum Normieren von Sequenzierungsdaten.

10. Verfahren zum Bestimmen der absoluten Menge einer oder mehrerer Zielsequenzen in einer Probe, insbesondere in einer Zell-, Gewebe- oder Organprobe, unter Verwendung eines Sets nach einem der Ansprüche 1 bis 9.

11. Verfahren zum Bestimmen von Referenzwerten bei der Nukleotidsequenzierung, umfassend die Schritte
- Zusetzen eines Sets nach einem der Ansprüche 1 bis 9 zu einem Gemisch von Zielsequenzen, wodurch eine Bibliothek von Nukleinsäuremolekülen erzeugt wird,
- Ligieren von Adaptern an die Bibliothek,
- gegebenenfalls Amplifizieren der Bibliothek,
- Durchführen eines Nukleotidsequenzierungsverfahrens,
- Bestimmen der Menge an Nukleinsäuremolekülen jedes Teilsets als Referenzwert.

12. Verfahren nach Anspruch 11, wobei die Kopienzahl von kleinen RNA-Molekülen aus verschiedenen Zelltypen verglichen wird.

13. Verfahren zum Bestimmen der Anzahl von Nukleinsäuremolekülen in einer Probe, umfassend die Schritte
a) Zusetzen eines Sets nach einem der Ansprüche 1 bis 9 zu der Probe, um ein Gemisch von Nukleinsäuremolekülen zu erhalten, wodurch eine Bibliothek von Nukleinsäuremolekülen erzeugt wird,
b) gegebenenfalls Amplifizieren der Bibliothek,
c) Durchführen einer Sequenzierung der nächsten Generation der Bibliothek, die zu RNA-Sequenz-Reads von den Nukleinsäuremolekülen führt,
d) Bestimmen der Anzahl der Reads aus dem Set und aus der Probe.

14. Verwendung des Verfahrens nach einem der Ansprüche 10 bis 13 zum Beurteilen von Klonierungsverzerrungen, die während der Herstellung einer sRNA-Bibliothek auftreten.

15. Oligonukleotid der allgemeinen Formel:
p-(N)ₘ(x)(N)ₘ-2'-O-Methyl,
wobei
N ein Zufallsnukleotid von beliebigen von A, U, G, C ist;
X eine Kernsequenz ist, die mindestens zwei Fehlpaarungen verglichen mit einer Zielsequenz mit einer Länge von 8, 9, 10, 11, 12, 13, 14, 15, 16, 17 oder 18 Nukleotiden, die beliebige von A, U, G, C umfassen, enthält;
m 3, 4 oder 5 ist.

## Revendications

1. Ensemble comprenant au moins deux sous-ensembles de molécules d'acide nucléique simple brin, chaque molécule d'acide nucléique comprenant dans le sens 5' à 3' :
a) un phosphate en 5',
b) une séquence d'au moins 3 nucléotides aléatoires,
c) une séquence noyau d'au moins 8 nucléotides, laquelle séquence contient deux mésappariements ou plus comparativement à une séquence cible,
d) une séquence d'au moins 3 nucléotides aléatoires, et
e) une modification en 3',
dans lequel chaque sous-ensemble comprend une pluralité de molécules d'acide nucléique comportant une séquence nucléotidique noyau identique et différents nucléotides aléatoires, et
dans lequel les molécules d'acide nucléique de chaque sous-ensemble différent d'au moins un nucléotide de la séquence nucléotidique noyau.

2. Ensemble selon la revendication 1, dans lequel la pluralité de molécules d'acide nucléique comprend des séquences nucléotidiques aléatoires contenant toutes des combinaisons de quatre nucléotides de A, C, G, U ou A, C, G, T.

3. Ensemble selon l'une quelconque des revendications 1 à 2, dans lequel la molécule d'acide nucléique est une molécule d'ARN, imitant spécifiquement un petit ARN, spécifiquement choisie dans le groupe constitué par un ARNsi, un ARNtasi, un ARNsn, un miARN, un ARNsno, un ARNpi et un ARNt et tout précurseur de ceux-ci.

4. Ensemble selon l'une quelconque des revendications 1 à 3, dans lequel la séquence nucléotidique noyau comprend de 8 à 25 nucléotides, de préférence de 10 à 20 nucléotides, de préférence de 12 à 18 nucléotides, de préférence 13 nucléotides.

5. Ensemble selon l'une quelconque des revendications 1 à 4, dans lequel la séquence de nucléotides aléatoires comprend de 3 à 7 nucléotides, de préférence de 3 à 5 nucléotides, de préférence 4 nucléotides.

6. Ensemble selon l'une quelconque des revendications 1 à 5, dans lequel le phosphate en 5' est choisi dans le groupe d'un monophosphate, un diphosphate et un triphosphate et dans lequel la modification en 3' est choisie dans le groupe constitué par une 2'-O-méthylation [groupe 2'-O-méthyle] et une hydroxylation [groupe hydroxy].

7. Ensemble selon l'une quelconque des revendications 1 à 6, dans lequel les sous-ensembles sont présents en une quantité de 1 à 10000 amol, de préférence de 10 à 5000 amol, comprenant spécifiquement différentes quantités de chaque sous-ensemble.

8. Ensemble selon l'une quelconque des revendications 1 à 7, dans lequel la séquence cible peut être toute séquence d'intérêt, spécifiquement un génome ou un transcriptome d'un organisme, une séquence provenant d'un virus, d'une bactérie, d'animaux, de plantes, spécifiquement une population d'ARN, de petits ARN, de petits ARN dynamiques.

9. Utilisation d'un ensemble selon l'une quelconque des revendications 1 à 8 sous la forme de sondes insérées pour normaliser les données de séquençage.

10. Procédé permettant de déterminer la quantité absolue d'une ou plusieurs séquences cibles dans un échantillon, spécifiquement dans un échantillon de cellule, de tissu ou d'organe à l'aide d'un ensemble selon l'une quelconque des revendications 1 à 9.

11. Procédé permettant de déterminer des valeurs de référence dans un séquençage de nucléotides, comprenant les étapes de
- ajout d'un ensemble selon l'une quelconque des revendications 1 à 9 dans un mélange de séquences cibles, générant ainsi une banque de molécules d'acide nucléique,
- ligature d'adaptateurs à la banque,
- amplification éventuelle de ladite banque,
- exécution d'un procédé de séquençage de nucléotides,
- détermination de la quantité de molécules d'acide nucléique de chaque sous-ensemble en tant que valeur de référence.

12. Procédé selon la revendication 11, dans lequel les nombres de copies de molécules de petits ARN provenant de différents types de cellules sont comparés.

13. Procédé permettant de déterminer le nombre de molécules d'acide nucléique dans un échantillon, comprenant les étapes de
a) ajout d'un ensemble selon l'une quelconque des revendications 1 à 9 à l'échantillon pour obtenir un mélange de molécules d'acide nucléique, générant ainsi une banque de molécules d'acide nucléique,
b) amplification éventuelle de ladite banque,
c) exécution d'un séquençage de nouvelle génération de ladite banque conduisant aux lectures de séquences d'ARN à partir desdites molécules d'acide nucléique,
d) détermination du nombre de lectures à partir de l'ensemble et à partir de l'échantillon.

14. Utilisation du procédé selon l'une quelconque des revendications 10 à 13 pour évaluer les écarts de clonage se produisant pendant la préparation d'une banque de sARN.

15. Oligonucléotide de formule générale :
p-(N)ₘ(x)(N)ₘ-2'-O-méthyle,
dans laquelle
N représente un nucléotide aléatoire parmi l'un quelconque de A, U, G, C ;
X représente une séquence noyau contenant au moins deux mésappariements comparativement à une séquence cible ayant une longueur de 8, 9, 10, 11, 12, 13, 14, 15, 16, 17 ou 18 nucléotides comprenant l'un quelconque de A, U, G, C ;
m vaut 3, 4 ou 5.


### SEQUENCE LISTING

<110> Gregor Mendel Institute of Molecular Plant Biology
<120> NOVEL SPIKE-IN OLIGONUCLEOTIDES FOR NORMALIZATION OF SEQUENCE DATA
<130> GM002EP
<160> 10
<170> Patent In version 3.5
<210> 1
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 1
   nnnngaguca ugcauuannn n 21
<210> 2
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 2
   nnnnuaugcc agaagucnnn n 21
<210> 3
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 3
   nnnnucuaac gugccuannn n 21
<210> 4
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 4
   nnnnugcgca gacauaannn n 21
<210> 5
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 5
   nnnnaagcga uuacgacnnn n 21
<210> 6
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 6
   nnnnauguga augaccgnnn n 21
<210> 7
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 7
   nnnnguaccc augugaannn n 21
<210> 8
   <211> 21
   <212> RNA
   <213> Artificial Sequence
<220>
   <223> oligonucleotide
<220>
   <221> misc_feature
   <222> (1)..(4)
   <223> N is any of A, U, G, C
<220>
   <221> misc_feature
   <222> (18)..(21)
   <223> N is any of A, U, G, C
<400> 8
   nnnnuauuga ucgcgcunnn n 21
<210> 9
   <211> 21
   <212> DNA
   <213> Artificial Sequence
<220>
   <223> adapter
<400> 9
   agatcggaag agcacacgtc t 21
<210> 10
   <211> 25
   <212> RNA
   <213> Artificial Sequence
<220>
   <223> adapter
<400> 10
   guucagaguu cuacaguccg acgau 25